# NOUVEAU FASCICULE DE BREVET EUROPEEN

(11) **EP 0 792 930 B2**
(45) Date de publication et mention de la décision concernant l'opposition: **16.12.2009**
(45) Mention de la délivrance du brevet: 28.08.2002
(21) Numéro de dépôt: 97870032.6
(22) Date de dépôt: 28.02.1997
(51) Int. Cl.: C12N 1/04, A21D 8/04

(54) **Procédé et installation de conditionnement et d'utilisation d'une crème de levure stabilisée**
Verfahren und Anlage zur Konditionierung und zur Verwendung stabilisierter Hefecreme
Process and system for conditioning and using stabilized yeast cream

(30) Priorité: 01.03.1996 BE 9600184
(43) Date de publication de la demande: 03.09.1997
(73) Titulaire: LESAFFRE et Cie, F-75001 Paris (FR); N.V. ALGIST-BRUGGEMAN, B-9000 Gent (BE)
(72) Inventeur: Van Eetvelde, Gabriel Petrus, 9440 Evergem (BE); Blomme, Karel Alfons Frans, 9440 Evergem (BE); Smet, Peter René Anna, 9100 Sintz-Niklaas (BE)
(74) Mandataire: Khairallah, Murielle

(56) Documents cités:
- EP-A- 0 461 725
- DE-C- 528 981
- FR-A- 1 384 258
- GB-A- 1 425 979
- DATABASE WPI Section Ch, Week 9025 Derwent Publications Ltd., London, GB; Class D11, AN 90-189410 XP002019477 & JP 02 124 054 A (KANEGAFUCHI CHEM KK) , 11 mai 1990

## Description

La présente invention est relative à un procédé de conditionnement et d'utilisation d'une crème de levure après une conservation tranquille et prolongée, sous forme d'une suspension stable.

On connaît par le document FR-A-1384258 qui date de 1963 l'utilisation d'une crème de levure non stabilisée par un additif. Cette crème de levure est transportée dans des réservoirs spéciaux sous une atmosphère de gaz de protection, en général du CO₂, elle est pompée ensuite dans des réservoirs de magasinage spéciaux sous pression de CO₂, munis d'un moyen de réfrigération autonome de la crème de levure, d'un moyen d'agitation de cette crème permettant une homogénéisation de la suspension de cellules de levures. Les moyens décrits dans ce document FR-A-1384258 sont complexes, coûteux, ils n'apportent qu'une solution partielle aux problèmes posés et ils n'ont fait l'objet à notre connaissance d'aucune réalisation pratique.

Depuis plusieurs années, dans les grands pays développés où il existe de grandes boulangeries industrielles, celles-ci sont livrées en camions-citernes par citernes complètes de l'ordre d'au moins 20 m³. Cette crème est pompée, puis stockée et utilisée dans des installations comprenant des bacs à crème d'au moins 20 m³, munis:
- d'une double enveloppe dans laquelle circule un liquide réfrigérant de manière à assurer le maintien de la crème à basse température.
- d'un système d'agitation permettant une homogénéisation de la crème de levure, et
- d'un dispositif de nettoyage avec nettoyage en place des bacs à crème.

Ces installations ont l'inconvénient d'être coûteuses, et posent dans la pratique beaucoup de problème d'asepsie et de nettoyage, problèmes qui demandent un personnel spécialisé. De tels investissements avec l'environnement qu'ils nécessitent ne sont envisageables que dans des grandes boulangeries industrielles ayant une consommation de crèmes de levures d'au moins un m³ par jour et de préférence plusieurs m³ par jour.

Le document EP-A-0 461 725 propose de supprimer l'agitateur mécanique et de le remplacer par une stabilisation de la crème par des stabilisants alimentaires classiques ajoutés en faible quantité, à savoir des gommes d'origine naturelle végétale ou microbienne de provenances diverses : gomme provenant de graines guar ou caroube, gomme adragante, arabique ou xanthane utilisée à raison de l'ordre de 0,03 à 1 % en poids par rapport à la crème de levure. L'emploi de gommes apporte seulement une solution à la stabilisation de la crème de levure, mais la seule suppression d'un agitateur n'apporte qu'une très faible économie aux installations décrites ci-dessus et notamment ne résout pas les problèmes liés aux risques de contamination lors du transport, de la livraison, et du stockage de la crème.

Le document EP-A-0 599 776 décrit quant à lui une installation de stockage classique mais miniaturisée, montée sur roues. Cette installation. de conception banale, comporte les équipements d'une installation fixe classique, à la seule différence que le nettoyage en place est réalisé chez le levurier et non chez le boulanger. En d'autres termes, l'installation chez le boulanger ne comporte pas de circuit avec un récipient contenant une solution de nettoyage pour le nettoyage de son installation. Cette formule est très coûteuse et fait notamment l'impasse sur les problèmes de nettoyage se posant lors de l'utilisation de la crème.

La présente invention vise un procédé de conditionnement et d'utilisation d'une crème de levure permettant de limiter, voire d'éviter tout problème de contamination de la crème de levure depuis son conditionnement en une quantité de 100 litres à 3000 litres, en général sur son lieu de fabrication dans la levurerie, jusqu'à son utilisation dans un pétrin, permettant d'éviter des transferts de crèmes de levure. Ce procédé assure une conservation des plus hygiéniques et ce pour de longues périodes de stockage, tout en ne nécessitant pas d'installations complexes et coûteuses.

Le procédé suivant l'invention est un procédé de conditionnement et d'utilisation d'une crème de levure après une conservation tranquille et prolongée, sous forme d'une suspension stable, dans lequel :
- on stabilise la crème de levure par l'emploi d'un additif alimentaire de famille des épaississants et stabilisants alimentaires;
- on conditionne la crème de levure stabilisée refroidie à moins de 10°C dans un conteneur propre et quasiment stérile, d'un volume compris entre environ 100 litres et 3000 litres, de préférence entre 200 litres et 3000 litres, muni d'un système formant évent;
- on place ledit conteneur, pendant tout le temps de son transport, pendant son stockage et pendant le soutirage de la crème de levure hors du conteneur pour l'utilisation de celle-ci, dans une enceinte dont on maintient la température à une température comprise entre -1°C et 10°C, de préférence entre 0 et 8°C, en particulier entre 0 et 5°C;
- on prélève, par gravité et/ou par une pompe volumétrique, la crème de levure au voisinage du fond du conteneur pour l'amener dans une ou des tuyauteries de distribution de crème de levure stabilisée pour son introduction dans un ou des pétrins; et
- on nettoie en place au moyen d'une solution de nettoyage au moins partiellement la ou les tuyauteries de distribution, après une période de temps déterminée.

Tous les paramètres du procédé indiqués ci-avant sont importants. La présence de l'additif alimentaire, la taille raisonnable du conteneur, l'évent permettant que le conteneur reste toujours à la pression atmosphérique et le moyen de prélèvement permettent d'assurer une composition de la crème stable et sensiblement constante lors de l'utilisation de la crème de levure du conteneur. L'évent permet en particulier un dégazage ou une élimination du gaz carbonique formé au cours de la conservation et retenu à l'intérieur de la crème stabilisée. Les dispositions d'ordre hygiéniques précédemment définies permettent d'éviter des problèmes de contamination et/ou de dégradation. Le conteneur utilisé est soit récupéré par la levurerie pour être nettoyé et réutilisé, soit jetable.

Ce procédé assure un stockage, une conservation et une utilisation hygiénique de la crème de levure. Il a l'avantage de permettre dans des conditions à la fois totalement hygiénique, la crème de levure étant un produit hautement périssable, et très économique, l'utilisation de crème de levure dans des boulangeries ayant des consommations de levures de panification réduites, de l'ordre de quelques dizaines de litres à quelques centaines de litres de crème de levure à environ 16-22 % de matières sèches.

De façon avantageuse, on conditionne, transporte, stocke et soutire la crème dans un conteneur en un matériau agréé pour le contact avec les produits alimentaires, équipé
- d'une tuyauterie de remplissage arrivant suffisamment à proximité du fond du conteneur de manière à ce que si la crème est mise en circulation fermée, il n'y ait pas formation de mousse,
- d'au moins une boule de nettoyage,
- d'une sonde de niveau,
- d'un système d'évent pour maintenir le conteneur à pression atmosphérique,
- d'une vanne de vidange de niveau bas.

Selon une forme de mise en oeuvre du procédé suivant l'invention, la tuyauterie de remplissage est sensiblement verticale et est perforée latéralement près du haut du conteneur de manière à pouvoir servir dans le système d'évent, lorsque le conteneur est rempli de crème de levure. Ladite tuyauterie présente en outre une ouverture située à une distance de moins de 10 cm, de préférence de moins de 5 cm du fond du conteneur. Avantageusement, le conteneur est équipé d'au moins une deuxième tuyauterie perforée latéralement près du haut du conteneur, de manière à pouvoir servir dans le système d'évent, lorsque la première tuyauterie est utilisée en circulation d'un liquide. L'évent en partie extérieure du conteneur peut par exemple être une crépine ou encore un tube en U.

Selon une autre forme de mise en oeuvre du procédé, le système d'évent est muni d'un filtre empêchant l'entrée de contaminants. Un tel filtre est par exemple un filtre présentant une porosité inférieure à 0,22 µm.

Selon une autre forme de réalisation possible du procédé suivant l'invention, on conditionne la crème de levure stabilisée dans une poche souple, de préférence jetable, autoportante ou de préférence sous forme d'une poche supportée par une armature recyclable. De façon avantageuse, le conteneur souple est équipé d'un système réutilisable formant évent et sonde de niveau, et il est conçu de manière à pouvoir être vidangé complètement et aisément.

La sonde de niveau du conteneur rigide ou souple est avantageusement une sonde de niveau bas de préférence reliée à une alarme de niveau bas ou à un système automatique prenant en compte ledit niveau bas.

Selon un détail d'une forme de réalisation avantageuse du procédé, on utilise une pompe volumétrique permettant une circulation lente de la crème et une circulation rapide de la solution de nettoyage.

De préférence, la pompe volumétrique peut être réglée pour avoir une vitesse de circulation de la crème de levure comprise entre 0,5 et 2 m/s, et par exemple une vitesse de circulation de la solution de nettoyage supérieure à 2 m/s.

Dans le procédé suivant l'invention, on peut utiliser en tant que pompe volumétrique, des pompes pneumatiques à membranes, des pompes à lobes, des pompes à vis excentrée ou queue de cochon. La sélection d'une pompe volumétrique est importante car les pompes centrifuges employées couramment dans l'industrie sont inadéquates.

Utilement, on place ledit conteneur muni d'un évent dans une enceinte dont on maintient la température à une température comprise entre -1°C et 10°C, de préférence entre 0 et 8°C, en particulier entre 0 et 5°C, l'évent du conteneur débouchant dans ladite enceinte.

De façon intéressante, on détecte au moyen d'un témoin la détérioration de la qualité boulangère de la crème de levure stabilisée.

Selon une forme de réalisation particulière du procédé, on utilise une pompe volumétrique pour le prélèvement de crème de levure hors du conteneur pour l'amener dans une boucle de circulation de crème de levure comprenant ladite pompe volumétrique, on prélève en un ou des points de la boucle de crème de levure pour alimenter un ou des pétrins, et on retourne la quantité de crème stabilisée non prélevée au susdit ou aux susdits points de prélèvement vers le conteneur. De préférence, dans cette forme de réalisation, on ramène la crème de levure dans le conteneur par une tuyauterie présentant une ouverture située au-dessus du fond de celui-ci, mais à une distance de moins de 10 cm, de préférence de moins de 5 cm dudit fond. Il est particulièrement avantageux dans ce procédé de refroidir la crème de levure au moyen d'un échangeur de chaleur, avant de ramener la crème de levure dans le conteneur.

Pour faciliter le nettoyage, chaque partie du circuit de la crème ou boucle de circulation est de préférence auto-vidangeable, c'est-à-dire chaque partie du circuit de la crème a un point bas par lequel elle peut être entièrement vidée.

Selon un détail d'une forme de mise en oeuvre du procédé, lors d'une étape de nettoyage, les tuyauteries de distribution de crème sont mises en circuit fermé sur un bac contenant une solution de nettoyage, cette dernière circulant grâce à la pompe volumétrique dudit circuit permettant de véhiculer la crème.

Un problème lié à l'emploi de la crème de levure stabilisée est dû au fait qu'elle peut contenir du gaz carbonique en sursaturation pouvant se libérer brutalement, ou des poches de gaz. Le dégagement de gaz carbonique à l'intérieur de la crème de levure dépend de la température de conservation. Il est donc avanvantageux de prévoir comme indiqué ci-dessus un témoin du respect de température ou un témoin de dégagement de gaz carboniques importants sont le signe d'une détérioration de la qualité boulangère de la crème. Il est aussi avantageux de prévoir des moyens de dégazage et de sélectionner une pompe pouvant véhiculer un tel liquide, ce qui est spécifiquement le cas des pompes volumétriques.

Dans le procédé selon l'invention, on utilise un additif alimentaire de la famille des épaississants et stabilisants alimentaires, en particulier un additif sélectionné dans la famille des homopolysaccharrides, éventuellement modifiés, et plus spécialement de préférence un additif sélectionné dans le groupe des amidons et des dérivés de la cellulose.

On peut par exemple utiliser une dispersion homogène d'un gel d'amidon modifié pour stabiliser la crème de levure.

A titre d'exemple, on peut utiliser en tant qu'agent stabilisant :
- 0,5 % à 2,0 % d'un amidon thermiquement et/ou chimiquement modifié, et de préférence entre 0,5 % et 1,5 % d'un amidon chimiquement modifié, et de préférence de l'adipate de diamidon acétylé répondant à la définition de l'amidon modifié E 1422 dans la nomenclature européenne, ledit stabilisant étant utilisé à raison de 0,5 % à 1,5 %, de préférence entre 0,8 % et 1,0 % en poids de la crème de levure, et/ou une dispersion d'un dérivé de la cellulose, de préférence du carboxyméthylcellulose, pour stabiliser la crème de levure, par exemple, un carboxyméthylcellulose haute viscosité, utilisé à raison de 0,1% à 0,3 % en poids de la crème de levure.

L'invention a encore pour objet un conteneur convenant pour la mise en oeuvre du procédé suivant l'invention, ledit conteneur réalisé en matériaux alimentaires et de manière à être aisément nettoyable, présentant :
- un volume compris entre 100 et 3000 litres,
- un système d'évent, tel que le conteneur reste toujours à la pression atmosphérique,
- une vanne située au voisinage du fond du conteneur pour permettre le soutirage de la crème de levure,
- au moins une boule de nettoyage, et
- une tuyauterie de remplissage du conteneur, ladite tuyauterie amenant la crème de levure au voisinage du fond du conteneur.
et dans lequel la tuyauterie de remplissage est sensiblement verticale, présente une ouverture située au-dessus dudit fond, à une distance de moins de 10 cm, de préférence de moins de 5 cm dudit fond, et comporte au moins une perforation latérale près du haut du conteneur de manière pouvoir servir d'évent.

Selon un exemple particulier de réalisation, le conteneur est muni:
- d'un détecteur de niveau minimal de crème de levure dans le conteneur, ledit détecteur émettant de préférence un signal d'alarme lorsque le détecteur détecte ledit niveau minimal,
- et/ou d'un témoin de la détérioration de la qualité boulangère de la crème de levure stabilisée présente dans le conteneur.

De préférence, le conteneur comporte au moins deux tuyauteries sensiblement verticales et perforées à proximité du haut. du conteneur, de manière à ce qu'au moins une tuyauterie soit libre pour être reliée à l'extérieur du conteneur et contribuer à former un évent, l'évent appartenant de préférence à l'un des types suivants : tube en U, crépine, filtre, filtre bactériologique.

L'invention a encore pour objet :
Une Installation pour le conditionnement et l'utilisation, sous forme d'une suspension stable, d'une crème de levure stabilisée par l'emploi d'un additif alimentaire de la famille des épaississants et stabilisants alimentaires après une conservation tranquille et prolongée, ladite installation comportant :
   - un conteneur (C) propre et quasiment stérile d'un volume compris entre environ 100 litres et 3000 litres et muni d'un système d'évent (9),
   - une enceinte (16) définissant une chambre contenant le conteneur (C), la température de ladite chambre étant maintenue à une température comprise entre -1°C et. 10°C,
   - une ou des tuyauteries de distribution (29), et
   - un ou des pétrins (34),
   l'installation permettant le prélèvement par gravité de la crème de levure stabilisée au voisinage du fond du conteneur (C) pour l'amener dans la ou les tuyauteries de distribution (29) pour son introduction dans le ou les pétrins (34),
   ladite installation permettant le nettoyage en place au moyen d'une solution de nettoyage au moins partiellement de la ou des tuyauteries de distribution (29).
   ainsi que
Une installation pour le conditionnement et l'utilisation, sous forme d'une suspension stable, d'une crème de levure stabilisée par l'emploi d'un additif alimentaire de la famille des épaississants et stabilisants alimentaires après une conservation tranquille et prolongée, installation comportant :
   - un conteneur (C) propre et quasiment stérile d'un volume compris entre environ 100 litres et 3000 litres muni d'un système d'évent (9);
   - une enceinte (16) définissant une chambre contenant le conteneur (C), la température de ladite chambre étant maintenue à une température comprise entre-1°C et 10°C,
   - une ou des tuyauteries de distribution (29),
   - une pompe volumétrique (36), et
   - un ou des pétrins (34),
   ladite pompe volumétrique permettant le prélèvement de la crème de levure stabilisée au voisinage du fond du conteneur (C) pour l'amener dans la ou les tuyauteries de distribution (29) pour son introduction dans le ou les pétrins (34),
   ladite installation permettant le nettoyage en place au moyen d'une solution de nettoyage au moins partiellement de la ou des tuyauteries de distribution (29).

L'invention a encore pour objet une installation pour la mise en oeuvre d'un procédé suivant l'invention, cette installation comprenant :
- une enceinte définissant une chambre adaptée pour contenir au moins un conteneur de crème de levure stabilisée, ladite enceinte étant munie d'un dispositif pour maintenir la température de la chambre entre -1°C et 10°C, de préférence entre 0 et 8°C, en particulier entre 0 et 5°C;
- une boucle de circulation pour amener la crème de levure vers un ou des points de la boucle où de la crème de levure peut être soutirée vers un ou plusieurs pétrins, ladite boucle se terminant par une canalisation permettant de ramener la crème de levure non prélevée au cours de son circuit dans le conteneur;
- une pompe volumétrique pour prélever de la crème de levure du conteneur et pour refouler la crème de levure dans la boucle de circulation, ladite pompe étant adaptée, d'une part, pour assurer la circulation de la crème de levure dans la boucle à une vitesse comprise entre 0,5 et 2 m/s de manière à éviter tout échauffement de la crème de levure, et d'autre part, pour assurer une circulation efficace d'une solution de nettoyage dans ladite boucle de circulation, et
- un réservoir pouvant être relié à la boucle de circulation par un ensemble de raccords mobiles ou tout autre dispositif prévu à cet effet de manière à permettre la circulation en circuit fermé d'une solution de nettoyage dans la boucle de circulation.

Avantageusement, la boucle de circulation de la crème de levure comprend un échangeur permettant le refroidissement de la crème de levure avant son retour dans le conteneur.

Dans une forme de réalisation possible, l'installation comprend un système d'arrêt automatique de la pompe lorsqu'un niveau minimal de levure dans le conteneur est détecté.

De préférence, pour l'installation suivant l'invention, chaque portion de la boucle de circulation de la crème de levure est auto vidangeable, c'est-à-dire qu'elle peut se vider complètement par gravité.

Selon une forme avantageuse de réalisation, l'enceinte est adaptée pour contenir deux conteneurs, un premier étant monté sur la boucle de circulation pour le soutirage de crème de levure, tandis que le deuxième est en réserve. Dans ce cas, il est possible de relier le premier conteneur et le deuxième conteneur par un système de raccords, de préférence souples, et de vannes, d'une part, à une canalisation alimentant la pompe volumétrique, et ,d'autre part, à une tuyauterie d'alimentation du conteneur recevant la quantité de crème non prélevée, et de commander par un système de commande lesdites vannes, de manière à ce que, lorsqu'un niveau minimal de crème de levure dans un conteneur est détecté, le système de commande manoeuvre lesdites vannes pour que l'autre conteneur soit mis en utilisation sur la boucle de circulation de la crème de levure. Les liaisons peuvent également être conçues de manière à ce que la crème de levure stabilisée résiduelle dans le conteneur en fin de soutirage puisse être transférée dans le second conteneur.

Selon une forme de réalisation préférentielle, la boucle de circulation est prévue de manière à être mise automatiquement en circulation soit sur le conteneur de crème de levure, soit sur le réservoir permettant le nettoyage en place.

Des caractéristiques et particularités de l'invention ressortiront de la description détaillée suivante dans laquelle il est fait référence aux dessins ci annexés qui montrent des formes de réalisation données à titre illustratif non limitatif.

Dans ces dessins :
- la figure 1 est une vue schématique d'une installation particulièrement simple et économique de boulangerie comprenant l'utilisation d'un conteneur souple jetable, suivant l'invention;
- la figure 2 est une vue de dessus d'un anneau de soutien d'un évent pour le conteneur souple montré dans la figure 1;
- la figure 3 est une coupe verticale partielle du conteneur souple de la figure 1 présentant l'anneau de soutien de la figure 2;
- la figure 4 est une coupe verticale partielle du conteneur souple de la figure 1, montrant l'anneau de soutien avec l'électrode de niveau et l'évent;
- la figure 5 est une vue de détail partiellement en coupe d'un conteneur rigide et réutilisable suivant l'invention correspondant au conteneur préféré;
- la figure 6 est une vue schématique d'une installation de boulangerie comprenant un conteneur rigide relié, par une boucle de circulation, à un dispositif de dosage;
- la figure 7 montre la circulation respectivement de la crème de levure et d'une solution de nettoyage dans une installation du type de la figure 6;
- la figure 8 est une vue schématique d'une autre installation de boulangerie pouvant être adoptée quand l'enceinte de la chambre froide est proche des pétrins, et
- la figure 9 est une vue schématique d'un détail d'une autre installation de boulangerie reliant deux conteneurs au système.

Dans ces diverses figures, les mêmes signes ou numéros de référence désignent des éléments identiques ou analogues.

Le conditionnement tranquille d'une crème de levure pendant plusieurs jours dans un conteneur dépourvu d'une agitation requiert que la crème de levure soit stabilisée à l'aide d'un agent épaississant uniformément réparti dans toute la masse de la crème de levure.

Il existe plusieurs moyens de déterminer l'homogénéité d'une crème de levure. Dans les tests décrits ci-dessous la matière sèche et la densité de la crème de levure sont comparés à des valeurs mesurées à des niveaux arbitrairement choisis en haut et en bas d'un récipient.

Dans les formes de réalisation particulières de crème de levure stabilisée décrite ci-après, la crème de levure contient, en tant qu'agent stabilisant, de 0,5 à 2 % en poids d'un amidon thermiquement et/ou chimiquement modifié ( de préférence un amidon thermiquement et chimiquement modifié ), en particulier de 0,8 à 1,5 % d'un amidon chimiquement modifié, tel que l'adipate de diamidon acétylé répondant à la définition de l'amidon modifié E1422 dans la nomenclature européenne, ce composé étant utilisé à raison de 0,8 à 1,5 % en poids du volume de la crème, c'est-à-dire 0.8 à 1,5 kg d'amidon modifié par 100 litres de crème de levure.

Un dérivé de la cellulose particulièrement intéressant est un carboxyméthylcellulose de haute viscosité.

Les amidons sont des épaississants alimentaires classiques, mais guère des stabilisants au sens strict de ce terme. Leur emploi pour stabiliser les crèmes de levure pouvait sembler d'autant plus contre-indiqué que les amidons peuvent servir de substrats à certaines bactéries amylolytiques éventuellement contaminantes. En sélectionnant comme agents épaississants de la famille des amidons, les amidons thermiquement et/ou chimiquement modifiés, en particulier un adipate de diamidon acétylé, il a été possible de manière surprenante d'obtenir une parfaite stabilisation de la crème de levure sur une longue période de temps, tout en évitant le risque de contamination par des bactéries amylolytiques. Des résultats intéressants de stabilisation ont également pu être obtenus au moyen d'un amidon prégélatinisé non modifié chimiquement, séché au laminoir, et présentant une teneur en amylopectine de 70%. Cet amidon prégélatinisé est utilisé à raison de 20g par litre de crème de levure.

Dans les exemples suivants, toutes les concentrations de stabilisants sont exprimées en % en poids / volume, c'est-à-dire en kg par 100 litres de crème de levure.

### EXEMPLES DE CREMES DE LEVURE STABILISEES

### Exemple 1 : Stabilisation d'une crème de levure par 1 % d'amidon modifié du type E1422

### 1. Préparation du gel

Un gel est préparé en ajoutant 9,2 kg d'amidon thermiquement et/ou chimiquement modifié du type E1422 à 120 l d'eau. Cet amidon modifié E1422 est un adipate de diamidon acétylé ayant les caractéristiques suivantes l'amidon avant modification est un amidon de maïs séché au laminoir contenant 99% d'amylopectine. Le mélange est agité pendant 30 minutes dans un mixeur industriel (1420 rpm. 2,2 kW). Le gel bien homogène est ensuite mélangé à la crème de levure dans les proportions suivantes : 26 l de gel (contenant environ 2 kg d'amidon modifié) dans 174 l de crème de levure et remué pendant 30 min. dans le même mixeur, afin d'obtenir une concentration finale de 1 % d'amidon E1422. Le gel et la crème de levure avant leur mélange avaient une température de moins de 10°C.

La crème de levure stabilisée est conservée à 4°C; des mesures de température sont faites chaque jour. Il est impératif que la chaîne de froid ne soit jamais interrompue : la température doit toujours être maintenue en dessous de 10°C, de préférence en-dessous de 8°C, en particulier en-dessous de 5°C.

### 2. Matière sèche et densité

La teneur en matière sèche des échantillons prélevés dans la couche supérieure et la couche inférieure d'un conteneur de 200 litres a été évaluée chaque semaine pendant un mois. Les résultats des mesures sont comparés à ceux d'un témoin non-stabilisé.

Le tableau 1 donne une évolution de la matière sèche (en g/l) de la crème de levure au cours de 4 semaines de conservation à 4°C.

**Tableau 1**

| Teneur en matière sèche d'une crème de levure stabilisée | | | | |
|---|---|---|---|---|
| | 1 % E1422 | | Témoin | |
| | En dessous | Au-dessus | En dessous | Au-dessus |
| fraîche | 19,2 | 19,2 | 18,8 | 14,8 |
| 1 semaine | 18,6 | 18,6 | 19,1 | 17,5 |
| 2 semaines | 18,4 | 18,4 | 19,4 | 15,5 |
| 3 semaines | 18,1 | 18,1 | 19,9 | 15,3 |
| 4 semaines | 17,9 | 17,9 | 20,5 | 14,8 |

Les résultats du tableau 1 ne montrent aucune différence de teneur en matière sèche entre les couches inférieures et supérieures de la crème stabilisée, au cours des quatre semaines de conservation. Il y a une corrélation directe entre la teneur en matière sèche et la densité car aucune variation de densité n'a été constatée entre les couches inférieures et supérieures de la crème stabilisée. La légère baisse de concentration en matière sèche au cours du temps est due à la consommation par la levure de ses sucres de réserve et à leur transformation en CO₂. La crème de levure stabilisée reste parfaitement homogène. Au bout des quatre semaines, le conteneur est vidé et on ne constate aucune formation de dépôt.

### 3. Pouvoir fermentatif

Des échantillons de levure stabilisée sont prélevés dans une couche du dessous et d'au-dessus d'un conteneur. Le pouvoir fermentatif est évalué par une mesure de la production de CO₂ sur deux heures, au moyen du fermentomètre de Burrows et Harrison publiée dans Journal of Inst. of Brewing, 1959, Vol. 65, p. 39-45, par S. Burrows et J.S. Harrison, selon le mode opératoire sur pâte sans sucre décrit dans le chapitre 6.1 de l'ouvrage "Guide pratique d'analyses dans les industries des céréales", GODON, B. et LOISEL, W. (Coord.) 1984, Technique et Documentation Lavoisier, p. 454-459.

L'ajout d'une quantité d'amidon modifié d'environ 1 % n'influence pas la qualité de la crème de levure. Après une semaine de conservation à 4°C, le pouvoir fermentatif d'une crème de levure stabilisée comme décrit ci-dessus est de 138 ml alors que celui du témoin non stabilisé, conservé dans les mêmes conditions mais homogénéisé, est de 135 ml.

On ne constate aucune différence dans la qualité des échantillons de crème de levure pris dans la couche supérieure et la couche inférieure (Tableau 2).

**Tableau 2 :**

| Evolution du pouvoir fermentatif de la crème stabilisée en ml de CO₂. | | |
|---|---|---|
| | fermentomètre | |
| | bas | haut |
| fraîche | 140 | 140 |
| 1 semaine | 138 | 138 |
| 2 semaines | 139 | 139 |
| 3 semaines | 137 | 137 |
| 4 semaines | 134 | 134 |

### 4. Essai de panification

Un essai de panification est effectué dans un fournil pilote.

A cet effet, une quantité déterminée de pâte est préparée en boulangerie selon la recette suivante :

| | |
|---|---|
| farine | 2350 g |
| eau | 1296 g |
| crème de levure | 71 g |
| sel | 47 g |
| matière grasse | 47 g |
| TOTAL | 3811 g |

La pâte est pétrie sur un pétrin spirale de marque OASE ® Spiral Kneter SPK8 pendant 3 minutes à une vitesse de rotation inférieure du pétrin correspondant à graduation 1 du sélecteur de vitesse du pétrin. Elle est ensuite pétrie pendant 4 minutes à une vitesse de rotation supérieure correspondant à la graduation 2.

La quantité de pâte obtenue est ensuite divisée en quatre parts égales. Une première part est utilisée pour mesurer le temps de levée T à 35°C nécessaire pour atteindre une certaine hauteur, ce temps est mesuré et correspond au premier chiffre exprimé en minutes. Cette pâte ayant une fermentation de temps T (minutes) à 35°C et à 80% d'humidité relative est cuite, le volume du pain obtenu est exprimé en litres et donne le second chiffre du tableau 3. Les trois autres parts sont cuites au four après une levée standard de 65 minutes à 35°C et à une humidité relative de 80%. Le volume de ces trois pains après cuisson est mesuré et leur moyenne est notée, cette moyenne forme le troisième chiffre du tableau 3.

**Tableau 3 :**

| Temps T pour obtenir une levée d'une certaine hauteur / volume d'un pain cuit après une levée d'un temps T / volume moyen d'un pain cuit après une levée de 65 minutes. | | |
|---|---|---|
| | Boulangerie | |
| | bas | haut |
| fraîche | 64/4.23/4.31 | 64/4.23/4.31 |
| 1 semaine | 65/4.23/4.25 | 67/4.29/4.23 |
| 2 semaines | 66/4.28/4.30 | 66/4.29/4.29 |
| 3 semaines | 66/4.38/4.36 | 67/4.34/4.31 |
| 4 semaines | 68/4.23/4.16 | 69/4.10/4.11 |

### 5. Bactériologie

Les résultats bactériologiques expriment le dénombrement total de micro-organismes à 35°C (PCA - Norme ISO 4833) et de coliformes à 30°C (VRBA - Norme ISO 4832).

**Tableau 4 :**

| Evolution des résultats bactériologiques | |
|---|---|
| semaine | PCA/VRBA |
| fraîche | <100/<10 |
| 1 semaine | <100/<10 |
| 2 semaines | <100/<10 |
| 3 semaines | <100/<10 |
| 4 semaines | <100/<10 |

Les résultats du tableau 4 indiquent une fabrication et une conservation hygiénique de la crème de levure.

### Exemple 2 : Stabilisation d'une crème de levure au moyen de carboxyméthylcellulose (E466)

### 1. Préparation du gel

La carboxyméthylcellulose ou CMC (E466) est une molécule connue pour ses caractéristiques viscosantes. Les essais ont été faits avec du CMC de haute viscosité dont les caractéristiques sont les suivantes :
- degré de substitution 0,8-0,95;
- granulométrie 80% en poids des particules ont un diamètre inférieur à 75 µm et moins de 0,5% en poids des particules ont un diamètre supérieur à 250µm;
- viscosité à 1% sur matière sèche de 3500 à 5500 mPa.s à 25°C mesurée sur Brookfield LVT 4/30.

Le CMC est ajouté directement à la crème à raison de 0,3 %. Le mélange (400g de crème) est remué pendant 30 minutes par agitation magnétique (1500rpm-1500 tours par minute).

### 2. Concentration utile de CMC (E466) haute viscosité

Les concentrations intéressantes sont comprises entre 0,1 % et 0,3 % (poids/volume) pour stabiliser la crème.

La crème stabilisée est alors conservée à 4°C. Il est impératif que la chaîne de froid ne soit jamais interrompue.

### 3. Matière sèche et densité

Comme dans l'exemple 1, la teneur en matière sèche d'échantillons prélevés dans la couche supérieure et la couche inférieure d'un conteneur de 200 l a été évaluée chaque semaine pendant trois semaines.

**Tableau 5 :**

| Teneur en matière sèche d'une crème de levure stabilisée au moyen de 0,3 % de E466 | | |
|---|---|---|
| 4°C | CMC E466 | |
| | bas | haut |
| fraîche | 22,0 | 22,0 |
| 1 semaine | 20,9 | 20,8 |
| 2 semaines | 20,5 | 20,6 |
| 3 semaines | 20,6 | 20,4 |

On ne constate aucune différence par rapport à l'exemple 1, en ce qui concerne la teneur en matière sèche et la densité des échantillons entre le bas et le haut du conteneur.

Les contrôles de l'activité fermentative de cette crème de levure, les tests de panification et les contrôles de la contamination bactériologique donnent des résultats entièrement satisfaisants, comme ceux de l'exemple 1.

### EXEMPLES D'INSTALLATION

### Premier exemple d'installation de boulangerie mettant en oeuvre un conteneur souple échangeable ou jetable

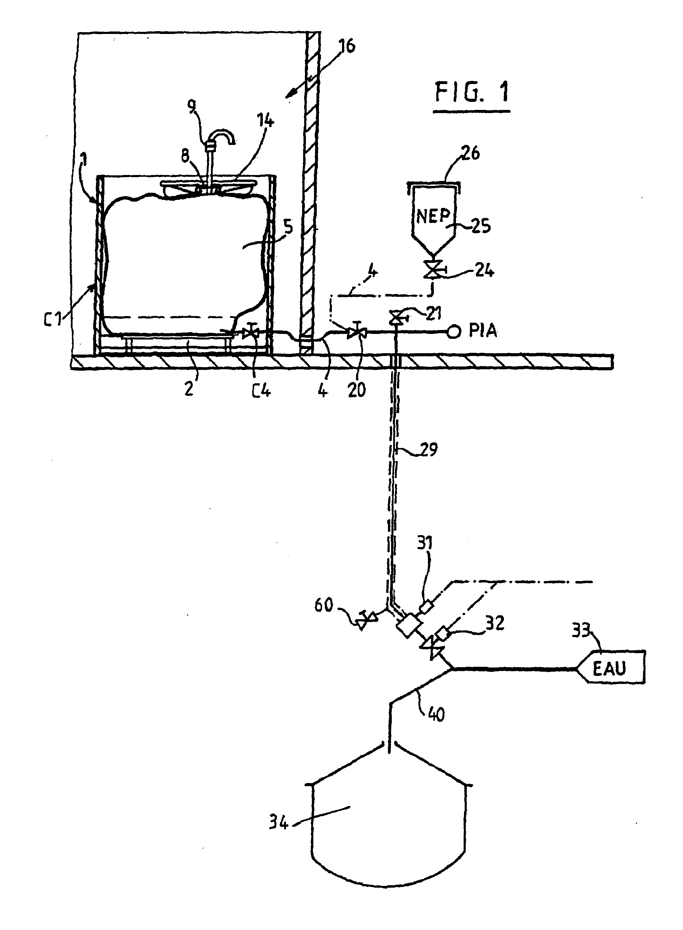

La figure 1 montre schématiquement une installation de boulangerie mettant en oeuvre un conteneur souple échangeable C . constitué d'un sac 5 éventuellement jetable en polyéthylène ou autre, monté dans un caisson 1 à ridelles pleines, porté par une euro-palette 2 à quatre entrées adaptées aux fourches d'un élévateur. Le conteneur est placé dans une chambre froide 16 dont la température est maintenue à 4°C par un dispositif non représenté, cette chambre froide est en élévation, et à distance faible du ou des pétrins. Le volume utile du sac peut être de 100, 250, 500,750 ou 1000 litres. Ce sac comporte deux manchons fermés par deux bouchons, un manchon et un bouchon sur le haut pour le remplissage, et un manchon et un bouchon bas ,permettant sa vidange complète, bouchon qui peut être remplacé par une vanne comme par exemple une vanne papillon.

La crème de levure est soutirée par gravité par le biais de l'orifice bas du containeur sur lequel est montée une vanne papillon C4 (point de raccordement pour le flexible 4), d'un tuyau flexible 4 et d'une vanne manuelle de soutirage 20 vers un appareil de mesure du débit de la pression PIA (Pressure Indication and Alarm) capable de détecter un niveau minimal et maximal de crème de levure dans le sac 5. La crème de levure est ensuite amenée par une conduite fixe 29 vers un point de dosage pourvu d'une vanne de vidange manuelle 60, d'un débitmètre électromagnétique 31, d'une vanne électromagnétique de dosage 32 et d'une conduite 40 pour amener la crème de levure au pétrin 34, cette conduite s'étendant entre la vanne électromagnétique de dosage 32 et le pétrin 34. Les dernières traces de crème de levure présente dans la conduite 40 sont récupérées et amenées par un courant d'eau de préparation provenant d'une source 33 et introduit directement en aval de la vanne électromagnétique de dosage 32 dans la conduite 40 (figure 1). Dès que les quantités requises de crème de levure et d'eau sont dosées, le pétrin 34 est mis en marche.

Une vanne 21 est montée sur la conduite 29, ladite vanne 21 se trouvant à un niveau supérieur au niveau de la vanne C4. Cette vanne 21 en position ouverte peut servir pour dégazer le gaz produit par la crème dans la conduite 29, c'est-à-dire servir d'évent.

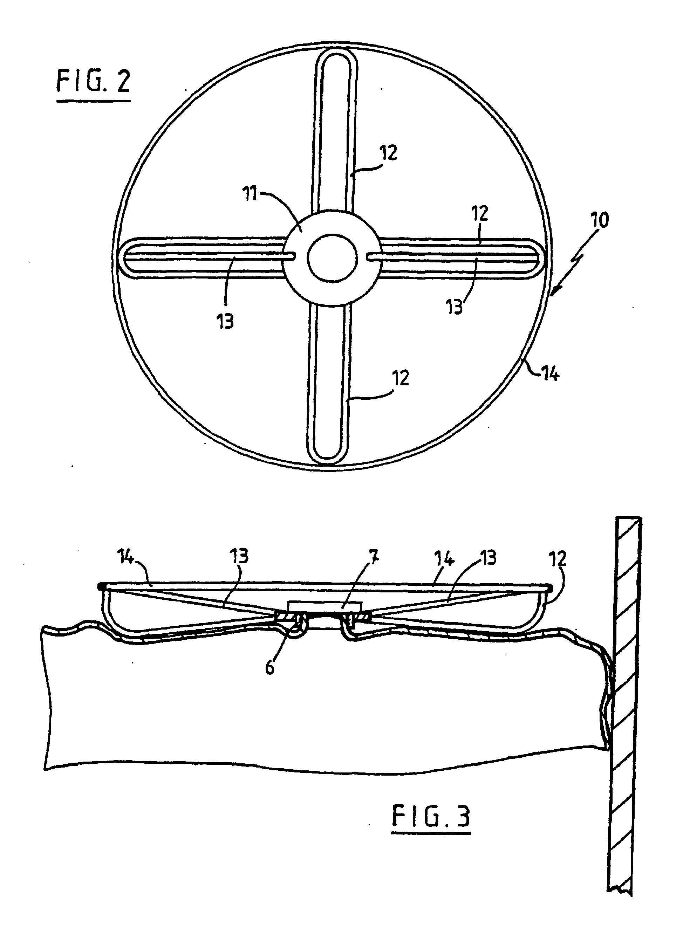

Au cours du transport et du stockage, le manchon 6 présentant un filetage extérieur qui forme l'ouverture de remplissage du conteneur souple est munie d'un bouchon 7 (figure 3). En service, ce bouchon 7 est remplacé par un bouchon de matière synthétique 8 traversé par un purgeur ou évent 9 présentant un coude d'évacuation replié sur lui-même pour prévenir l'introduction de poussière. Le bouchon de remplacement 8 est monté sur l'embouchure haute 6 du conteneur.

Un anneau de soutien 10 stabilise l'ouverture de remplissage et donc après vissage du bouchon 8, ledit bouchon et l'évent 9. L'anneau de soutien 10 comprend un disque de montage central 11 avec une structure filetée rigide destiné à être vissé sur le manchon 6. Quatre pattes 12 en forme de U, dont la courbe du U est pliée vers le haut, sont montés sur le disque 11. Les extrémités courbes extrêmes des pattes 12 sont reliées entre elles par un anneau extérieur 14. Des tiges obliques 13 s'étendent entre le disque central 11 et le point extrême de la courbe en U tournée vers le haut de pattes 12 pour rendre l'anneau de soutien 10 indéformable (figures 2 et 3).

Pour le nettoyage de la conduite 29 et du tuyau flexible 4, le tuyau flexible de prélèvement 4 est détaché de la partie inférieure du conteneur et relié à une vanne 24 du réservoir 25 de nettoyage en place (N.E.P.) pourvu d'un couvercle 26.

L'installation selon l'invention telle que réalisée dans cet exemple et schématisée dans la figure 1 correspond à des coûts de réalisation et de fonctionnement extrêmement faibles, elle est particulièrement adaptée à des boulangeries de capacité relativement restreinte.

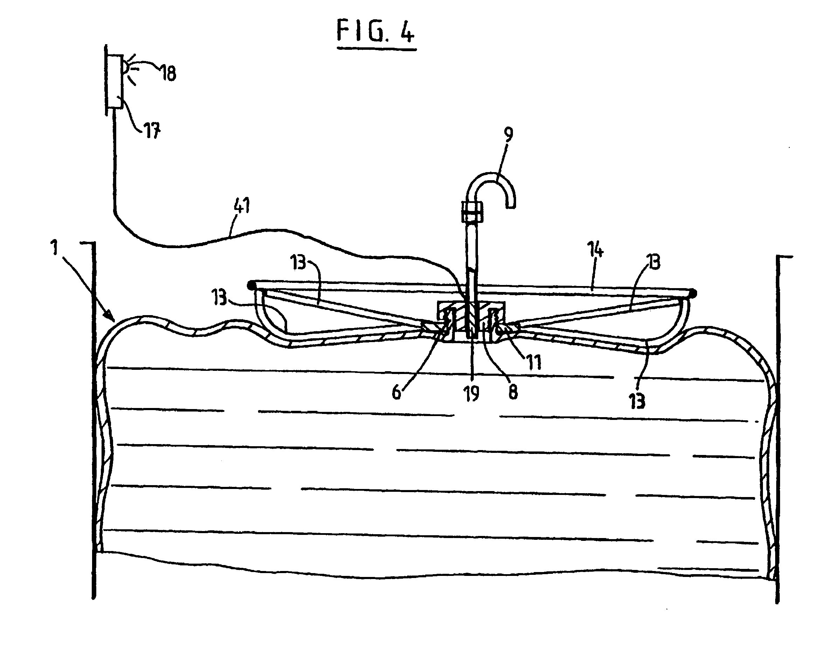

La figure 4 montre une forme de réalisation particulière d'un conteneur souple apte à être utilisé dans l'installation décrite ci-avant. Ce conteneur est similaire à celui décrit aux figures 1 à 3, si ce n'est qu'il est muni d'une électrode de niveau 19 indiquant le dernier dosage possible, et d'un dispositif 17,18 émettant un signal d'avertissement ou d'alarme.

Dans cette forme de réalisation, la partie inférieure du tube formant le purgeur ou l'évent 9, est constituée par une structure tubulaire 15 faisant office d'électrode à conducteur unique, qui est montée dans le bouchon de remplacement 8. Un fil conducteur souple 41 connecte la structure tubulaire 15 à un amplificateur 17 apte à activer un signal lumineux d'avertissement 18. L'électrode forme une jauge de niveau reliée à une alarme. Cette jauge permet de détecter un niveau minimal et/ou un niveau maximal et peut être montée soit dans un sac jetable, mais pourrait également être monté soit dans un conteneur rigide, en plastique ou en métal.

De manière intéressante, ces conteneurs qui doivent être maintenus à moins de 10°C peuvent être munis d'un indicateur avertissant si la qualité de la crème a pu subir une détérioration. Un tel indicateur peut être par exemple de nature thermique de manière à révéler les échauffements éventuels de la crème ou encore être un dispositif capable de détecter un excès de dégagement de CO₂ (sonde de mesure du gaz carbonique, réactif absorbant le gaz carbonique avec changement de couleur).

### Deuxième exemple d'installation de boulangerie comprenant un conteneur rigide relié à un dispositif de dosage avec une boucle de circulation

Le conteneur C, qui sert à transporter et à conserver la crème de levure stabilisée, est constitué d'un récipient rigide en matière synthétique alimentaire ou métallique permettant un nettoyage facile. Il est monté sur un socle de base muni d'entrées permettant sa manutention aisée par un élévateur. Ce récipient est non calorifugé et a une contenance de 500 à 3000 litres. Il est par exemple réalisé en polyéthylène ou en acier inoxydable, tel qu'en acier inox 304. Dans une forme de réalisation possible, le récipient aurait pu être placé sur un socle ou plateau muni de roues libres.

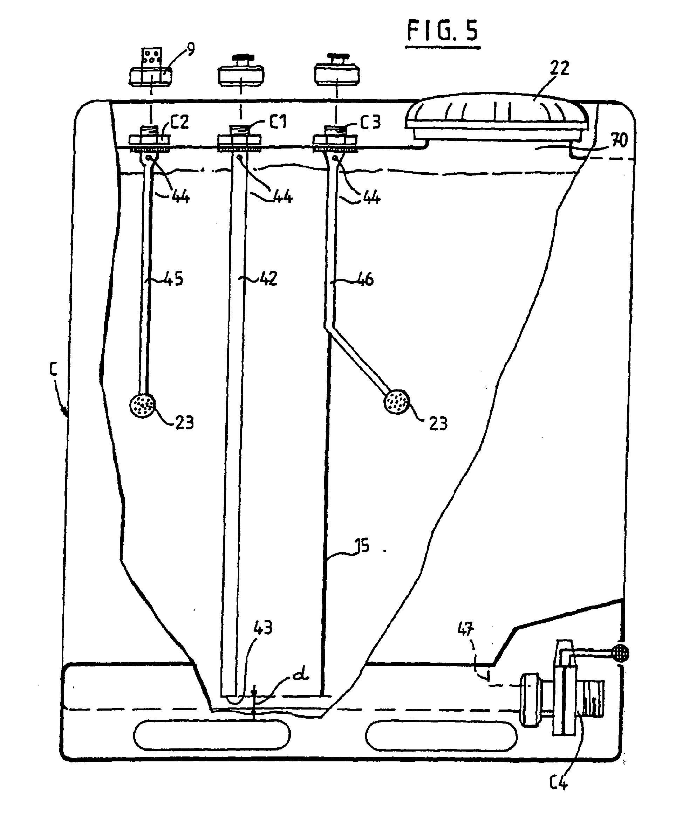

Dans l'exemple décrit ci-après, le conteneur C réalisé en polyéthylène alimentaire a une capacité utile de 1000 litres et l'ensemble de ces équipements sont réalisés en acier inox 304. Le détail de sa réalisation est donné à la figure 5. Ce conteneur C est équipé des accessoires suivants :
- un tube vertical 42 traversant le toit du conteneur et s'étendant jusqu'au voisinage du fond (la distance d entre l'ouverture inférieure 43 du tube 42 et le fond du conteneur est d'environ 3 cm), le tube 42 étant perforé de trous 44 juste en dessous du toit. L'extrémité du tube 42 située à l'extérieur du conteneur porte un raccordement C1.
- un tube 45 traversant le toit, dont l'extrémité située dans le conteneur porte une boule de nettoyage 23, ladite boule présentant une série d'orifices pour projeter dans toutes les directions la solution de nettoyage et/ou de rinçage, tandis que l'autre extrémité est munie d'un raccordement C2, le tube 45 étant perforé de trous 44 juste en dessous du toit .
- un tube 46 coudé dans le conteneur et traversant le toit, dont l'extrémité située dans le conteneur porte une boule de lavage 23, tandis que l'autre extrémité est munie d'un raccordement C3 muni d'un branchement, le tube 46 étant perforé de trous 44 juste en dessous du toit, une électrode 15 en acier inoxydable (inox 304) étant fixé sur ledit tube 46, ce dernier formant une partie de l'électrode, ladite électrode 15 servant à détecter un niveau minimal de crème de levure dans le conteneur C.
- une sortie 47 du conteneur C en point bas, cette sortie étant munie d'une vanne papillon manuelle d'un diamètre nominal de 50 mm, cette sortie formant le raccordement C4 pour soutirer par le bas du conteneur la crème de levure.
- une ouverture de visite 70 fermée par un couvercle 22 destinée à l'inspection du conteneur, notamment après son nettoyage.

Chaque passage d'un tube dans le conteneur est réalisé au moyen d'un raccord sanitaire.

Lors du transport, les raccordements C1,C2,C3,C4 sont fermés, étant entendu que l'un des raccordements C1, C2 ou C3 est fermé par une crépine 9.

Les tubes 42,45,46 sont perforés de trous 44, de sorte qu'une fois l'évent 9 placé sur l'un des raccordements C1,C2,C3, le conteneur est toujours à la pression atmosphérique. Avantageusement, l'évent peut être muni d'un filtre de manière à ce que l'air entrant dans le conteneur lors du soutirage de crème de levure soit rendu stérile. Le filtre a par exemple des pores de moins de 0,22 µm.

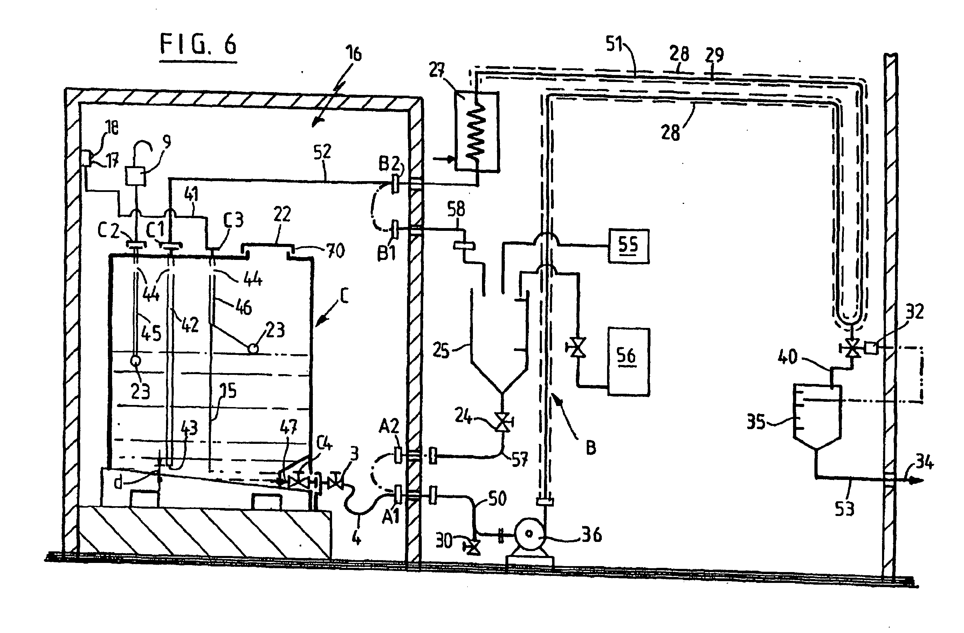

L'installation de la figure 6 comporte :
- une enceinte définissant une chambre froide 16 (température d'environ 4°C) destinée à recevoir un ou plusieurs conteneurs C,
- une boucle de circulation B pour relier le conteneur à un dispositif de dosage 32 et à un pétrin 34 d'un atelier de boulangerie, ledit atelier se trouvant à une distance non négligeable de la chambre froide 16, et
- un évent 9 monté sur le raccordement C2 du conteneur.

La boucle de circulation B comporte :
* un conduit flexible 4 reliant le raccordement C4 du conteneur au raccordement A1 du tuyau d'aspiration 50 d'une pompe volumétrique 36, ledit tuyau d'aspiration 50 étant muni d'une vanne de purge 30;
* la pompe 36 placée sous le niveau inférieur du conteneur, de manière à assurer l'amorçage de la pompe par effet gravitationnel;
* un conduit 29 dans lequel la pompe 36 refoule la crème de levure pour l'amener au dispositif de dosage 32, ledit conduit 29 élevant la crème de levure jusqu'à un niveau bien supérieur au toit du conteneur avant de la ramener à un niveau inférieur (niveau du conteneur), le conduit 29 étant muni d'un isolant thermique 28;
* un conduit 51, avantageusement muni d'un isolant thermique, s'étendant entre le dispositif de dosage 32 vers un échangeur tubulaire 27 pour refroidir la crème de levure à une température d'environ 5°C avant de la ramener vers le conteneur C, ledit conduit 51 amenant la levure non prélevée jusqu'à un niveau supérieur au niveau du conteneur C, cet échangeur tubulaire étant nécessaire lorsque la distance séparant le conteneur au pétrin est supérieure à 15m;
* l'échangeur tubulaire 27 situé à un niveau supérieur au conteneur C et relié audit conteneur par un conduit flexible 52, ledit échangeur étant agencé de manière à ce que la canalisation refroidie dans laquelle s'écoule la crème de levure présente toujours une inclinaison vers le bas, de manière à ce que la crème de levure s'écoule toujours vers le bas dans ladite canalisation et que ledit échangeur soit auto-vidangeable;
* le conduit flexible 52 présentant une première extrémité raccordée au raccordement B2 du conduit 51 et une deuxième extrémité raccordée au raccordement C1 du conteneur C pour ramener la crème de levure dans le bas du conteneur par le tube 42.

La boucle de circulation fermée B de crème peut servir de moyen pour assurer un dégazage de la crème, c'est-à-dire une élimination du gaz carbonique formé au cours de la conservation et retenu à l'intérieur de la crème. Bien entendu, ce dégagement de gaz carbonique dépend de la température de conservation de la crème de levure, et il est important de le limiter au maximum. Il peut être avantageux d'avoir un témoin de son importance.

L'électrode de niveau 15 est relié à un système d'alarme 17 émettant au moins un signal lumineux 18 et éventuellement un signal commandant l'arrêt de la pompe 36, lorsque le niveau de crème dans le conteneur est minimal, par exemple lorsque le niveau de crème dans le conteneur est inférieur au niveau de l'orifice 43 du tube 42 pour ramener la crème dans le conteneur C.

La pompe 36 est une pompe volumétrique qui permet de prélever de la crème de levure du conteneur et de refouler la crème de levure dans la boucle de circulation B, ladite pompe étant adaptée, pour assurer l'écoulement de la crème de levure dans la boucle à une vitesse comprise entre 0,5 et 2 m/s et pour éviter un échauffement de la crème de levure dû au frottement dans les tuyauteries. D'autre part, la pompe est à vitesse variable de sorte qu'elle est à même d'assurer la circulation d'une solution de nettoyage dans la boucle de circulation à une vitesse supérieure à 2 m/s, de manière à obtenir un nettoyage efficace.

La crème de levure prélevée par le dispositif de dosage peut être amenée, soit directement à un pétrin, soit dans un réservoir intermédiaire 35 par un conduit 40 avant d'alimenter plusieurs pétrins 34 par une conduite 53 munie de vannes d'alimentation 54 de crème aux pétrins.

Une amenée 33 d'eau non représentée à la figure 6 est prévue sur la conduite 40 juste en aval du dispositif de dosage pour s'assurer que la dose de crème mesurée par le dispositif 32 passe entièrement dans le réservoir intermédiaire 35 et de manière à pouvoir rincer ce circuit.

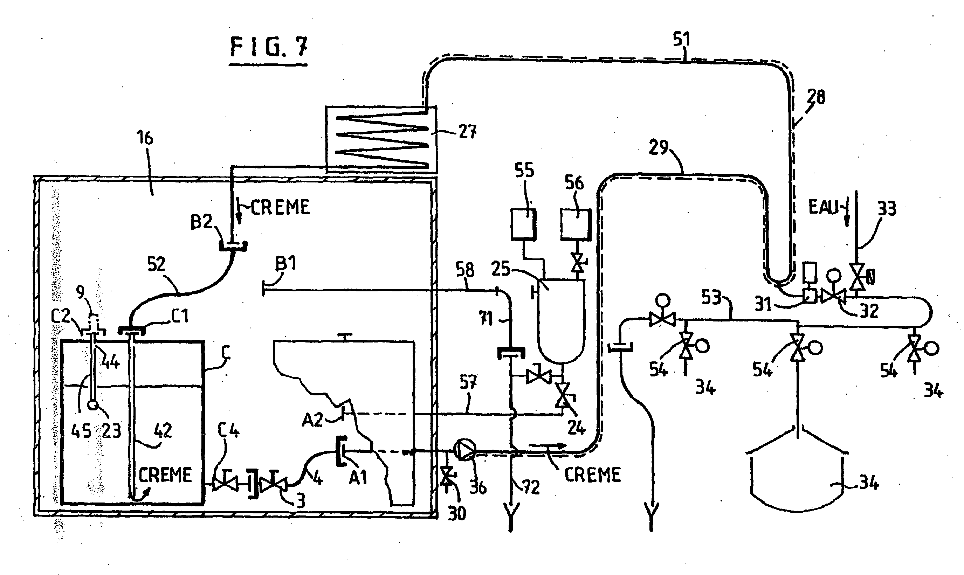

La figure 7 montre schématiquement la circulation de la crème de levure dans l'installation représentée à la figure 6 lors du soutirage de crème vers les pétrins 34. Les flèches EAU et CREME montrent respectivement la circulation ou le déplacement d'eau et de crème.

Dans ce schéma (figure 7), l'installation de dosage de la crème de levure dans les pétrins est réalisée grâce à un débitmètre électromagnétique 31 et des vannes électromécaniques automatisées 54 sans réservoir intermédiaire. Dans le cadre de ce schéma, la pompe 36 de préférence tourne en permanence et cette circulation permanente de la crème stabilisée permet son dégazage. L'enceinte 16 est prévue pour recevoir deux conteneurs, un premier en soutirage et recirculation, un second en réserve.

L'installation représentée à la figure 6 comporte également un dispositif de nettoyage de la boucle de circulation B. Ce dispositif comporte un réservoir 25 alimenté soit par une solution de nettoyage provenant d'une source 55 et/ou de l'eau provenant d'une source 56. Le réservoir 25 est relié via une conduite 57 munie d'une vanne 24 pour amener la solution de nettoyage à un point de raccordement A2 situé à l'intérieur de la chambre 16. Une conduite 58 s'étend entre un point de raccordement B1 situé dans la chambre 16 pour ramener la solution de nettoyage après passage dans la boucle de circulation B vers le réservoir 25.

L'opération de nettoyage en place s'effectue en 40 minutes environ. Un rinçage préliminaire à l'eau d'environ 5 minutes est suivi du nettoyage au moyen d'un produit nettoyant et désinfectant pendant environ trente minutes puis d'un rinçage final à l'eau pendant environ cinq minutes.

Pour la mise en oeuvre de l'opération de nettoyage de la boucle de circulation, la pompe 36 est mise à l'arrêt. Ensuite, la vanne 3 montée sur la conduite flexible 4 est fermée avant de débrancher le conduit flexible 4 du raccordement C4 du conteneur C et de le raccorder au point de raccordement A2. De manière similaire, le conduit flexible 52 est débranché du raccordement C1 du conteneur C pour le raccorder au point de raccordement B1 de la conduite 58 comprenant un conduit souple pouvant être raccordé au réservoir 25 ou à une tuyauterie 72 de rejet d'effluents vers l'égout (position représentée à la figure 7).

On remplit le réservoir 25 au moyen d'eau provenant de la source 55. On branche le conduit souple 71 sur la tuyauterie de rejet 73 à l'égout. On ouvre la vanne 24 et on met en marche la pompe 36. De cette façon, une première quantité d'eau du réservoir 25 rince toute la boucle de circulation y compris les conduits souples 52 et 4. De préférence on ouvre les vannes montées sur le conduit 53, de manière à permettre le rinçage du conduit 53. L'eau sortant du conduit 53 est alors amenée à l'égout. Cette étape de pré-rinçage est opérée pendant 5 minutes. On peut également effectuer un pré-rinçage prolongé de la boucle de circulation en branchant le conduit souple sur le réservoir 25. On arrête la pompe et on ouvre ensuite la vanne 30 pour purger la boucle de circulation. La boucle une fois purgée, on ferme la vanne 30 et on débranche le conduit souple 71 de la tuyauterie de rejeet 72 pour le brancher sur le réservoir 25. Avantageusement, la conduite 53 comporte une partie souple qui peut être débranchée de la canalisation de rejet et être branché sur un raccord du réservoir 25 pour permettre le nettoyage de la conduite 53 après l'ouverture de la vanne 32.

On prépare dans le réservoir 25 une solution de nettoyage à partir d'un désinfectant bactéricide tel que par exemple un alcalin chloré du type Neophoschloro provenant du réservoir 56 et d'eau provenant de la source 55.

Après ouverture de la vanne 24 permettant à la solution de nettoyage de s'écouler vers la pompe 36, la pompe est mise en fonctionnement de manière à assurer une vitesse de circulation de la solution dans la boucle B d'au moins 2 m/s. La solution pompée par la pompe 36 est refoulée dans la conduite 29 et dans la conduite 51 avant de retourner, via le conduit flexible 52 et la conduite 58, dans le réservoir 25. Après une circulation de la solution de nettoyage pendant environ 30 minutes, on évacue par la vanne de purge 30 la solution de nettoyage et on effectue enfin un dernier rinçage au moyen d'eau amenée dans le réservoir 25 pendant environ 5 minutes, ce rinçage étant effectué de la manière décrite ci-avant pur le pré-rinçage. Après ce demier rinçage, la vanne 30 permet de purger complètement la boucle de circulation B. Après cette purge, le conduit flexible 52 est branché sur le raccordement C1 d'un nouveau conteneur C, tandis que le conduit flexible 4 est branché sur le raccordement C4 dudit conteneur. On ouvre la vanne du raccordement C4 pour permettre à la crème de levure de s'écouler vers la pompe 36. La pompe 36 peut maintenant être mise en fonctionnement pour la circulation de crème de levure.

L'opération de nettoyage en place de la boucle de circulation est effectuée par exemple toutes les semaines. On peut également concevoir le système pour des nettoyages plus fréquents. Dans ce cas, on prévoit le stockage de la solution de nettoyage dans le bac 25 de manière à ce qu'elle puisse être recyclée et des rinçages où l'eau entre dans le circuit indépendamment du bac 25 et après circulation par la boucle à travers la pompe va à l'égout.

A titre d'exemple, le conteneur est transporté chez le client et utilisé pendant deux semaines, à raison de 5 jours par semaines, c'est-à-dire utilisation pendant 10 jours ouvrables à raison de 100 l/jour. La chaîne du froid est maintenue de manière ininterrompue pendant toute la période de stockage, de transport, de conservation et d'utilisation à une température inférieure à 10°C, en particulier à une température comprise entre 0 et 5°C. La crème après livraison chez un boulanger présentait lors de l'utilisation chez le boulanger les caractéristiques suivantes :

**Tableau 6 :**

| Evolution de la matière sèche et de la densité | | |
|---|---|---|
| Jour | Matière sèche | Densité |
| fraîche | 19,2 | 1,068 |
| 2 | 18,8 | 1,067 |
| 5 | 18,6 | 1,066 |
| 8 | 18,4 | 1,063 |
| 12 | 18,3 | 1,063 |
| 15 | 18,1 | 1,061 |

A part une perte en matière sèche et densité, due à la production de CO₂ (comme constaté dans l'exemple de crème de levure n° 1), la crème de levure reste homogène jusqu'à la fin des deux semaines de conservation.

**Tableau 7 :**

| Force fermentative et résultats de panification selon les méthodes décrites pour l'exemple de crème n°1 | | |
|---|---|---|
| Jour | Fermentomètre | Boulangerie |
| fraîche | 141 | 63/4.40/4.47 |
| 2 | 142 | 65/4.33/4.26 |
| 5 | 142 | 65/4.21/4.21 |
| 8 | 142 | 66/4.28/4.28 |
| 12 | 144 | 68/4.24/4.20 |
| 15 | 142 | 67/4.28/7.18 |

La crème est utilisée sans perte de force en boulangerie pendant 2 semaines; la perte de matière sèche n'a pas d'influence sur le pouvoir fermentatif (Tableau 7).

**Tableau 8 :**

| Bactériologie | |
|---|---|
| jour | (PCA/VRBA) |
| fraîche | <100/<10 |
| 7 | <100/<10 |
| 15 | <100/<10 |

La contamination bactériologique n'augmente pas pendant l'utilisation de la crème 15 jours en boulangerie.

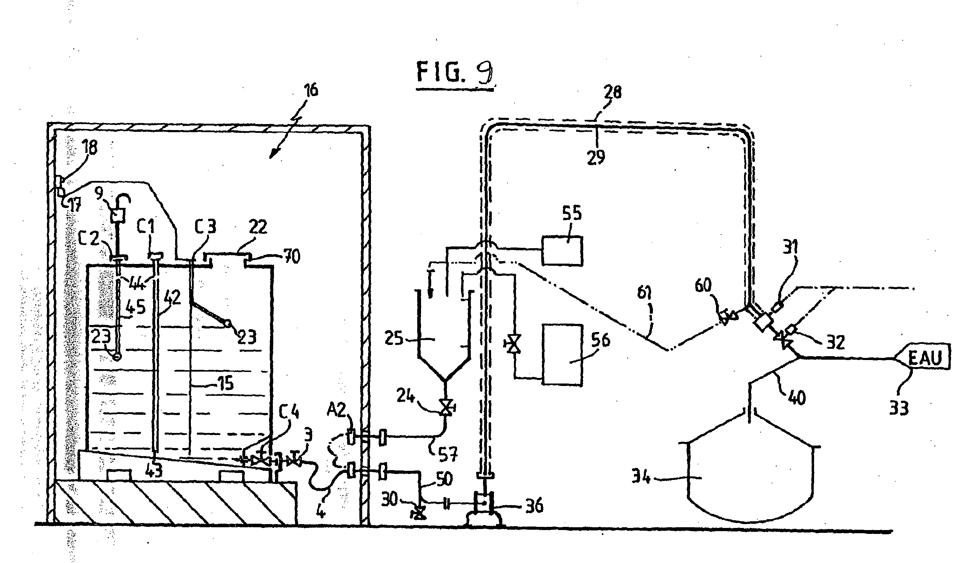

### Troisième exemple d'installation de boulangerie comprenant un conteneur rigide relié à un dispositif de dosage (Figure 9)

L'installation représentée à la figure 9 est similaire à celle de la figure 6, si ce n'est qu'elle ne comporte pas de conduite 51 pour retourner vers le conteneur C la crème non utilisée au dispositif de dosage 32 muni d'un débitmètre 31. La pompe 36 contrairement à l'exemple précédent ne marche pas en continu mais seulement pendant l'alimentation du ou des pétrins. Cette disposition n'est possible que lorsque la chambre froide et le ou les pétrins sont à faible distance l'un de l'autre. Pour le nettoyage, l'installation montrée à la figure 9 présente une vanne 60 montée sur la conduite 29 juste en amont du dispositif de dosage. Un conduit flexible 61 relie la vanne 60 au réservoir 25 pour la circulation de la solution de nettoyage dans la conduite 29 et dans la pompe 36.

### Quatrième exemple d'installations de boulangerie avec automatisation des circuits.

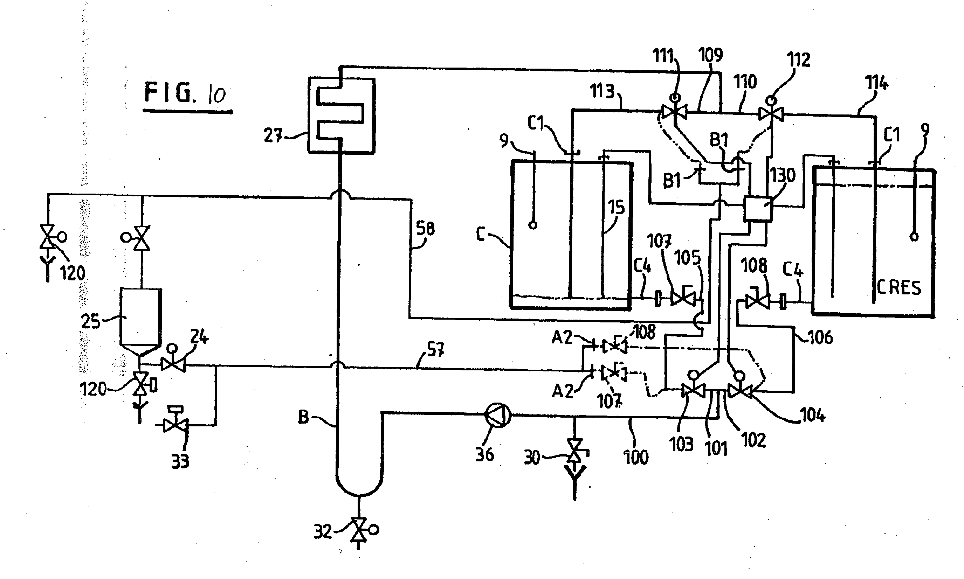

La figure 10 montre un détail d'une forme de réalisation d'une installation similaire à celle représentée à la figure 6. Selon ce détail, l'enceinte 16 est adaptée pour contenir deux conteneurs C.Crés., un premier C étant monté sur la boucle de circulation pour le soutirage de crème de levure, tandis que le deuxième Crés. est en réserve.

Dans cette installation, le premier conteneur C et le deuxième conteneur Crés. sont reliés par au moins une canalisation 100 à la pompe 36, ladite canalisation comprenant deux branches 101, 102 munies de vannes électrocommandées 103, 104 et par les tuyauteries souples 105, 106 munies de vannes manuelles 107, 108, lesdites tuyauteries pouvant être raccordées aux raccords C4 des conteneurs C et Crés.. La pompe 36 refoule dans la boucle de circulation B la crème de levure provenant soit du conteneur C lorsque les vannes 103 et 107 sont ouvertes, soit du conteneur Crés. lorsque les vannes 104 et 108 sont ouvertes. Avant de ramener la crème de levure non prélevée vers un conteneur, la crème de levure est refroidie dans l'échangeur 27. La boucle de circulation se termine par deux branches 109, 110 munies chacune d'une vanne électrocommandée 111, 112, et d'un conduit flexible 113, 114 destiné à être raccordé sur le raccord C1 respectivement du conteneur C et du conteneur Crés. En cours de fonctionnement, lorsque l'électrode 15 du conteneur C détecte un niveau bas dans le conteneur, un dispositif de commande 130 agit sur les vannes électrocommandées de manière à fermer les vannes 103, 111 et à ouvrir les vannes 112,104. Ainsi la pompe 36 prélève la crème de levure du conteneur Crés. tandis que la crème de levure non prélevée dans la boucle est renvoyée dans le conteneur Crés.. Dans une forme de réalisation possible, les vannes 111 et 112, 103 et 104 peuvent être remplacées par deux vannes à trois voies.

Pour le nettoyage de cette installation on débranche, après fermeture des vannes 107, 108, les conduits souples 105, 106 des raccordements C4 pour raccorder lesdits conduits sur deux branchements A2 d'un circuit de nettoyage. On débranche également les conduits souples 113, 114 des raccords C1 pour les raccorder ensuite sur les branchements B1 de la conduite 58. Après remplissage du réservoir 25 on ouvre les vannes 24, 107, 108, 103 et 104 et on actionne la pompe pour que cette dernière envoie la solution de nettoyage du réservoir 25 dans la boucle B, la solution de nettoyage sortant de ladite boucle étant ramenée par le conduit 58 dans le réservoir 25.

Ainsi qu'on le remarquera, l'installation est munie de plusieurs vannes de purge 30, 120 de manière à purger les différentes portions de l'installation. Par ailleurs, on pourrait également prévoir les vannes et raccordements nécessaires pour que les séquences de nettoyage puissent également être réalisées de manière automatique.

## Revendications

1. Procédé de conditionnement et d'utilisation d'une crème de levure après une conservation tranquille et prolongée, sous forme d'une suspension stable, dans lequel :
- on stabilise la crème de levure par l'emploi d'un additif alimentaire de la famille des épaississants et stabilisants alimentaires ;
- on conditionne la crème de levure stabilisée refroidie à moins de 10°C dans un conteneur propre et quasiment stérile (C), d'un volume compris entre environ 100 litres et 3000 litres, de préférence entre 200 litres et 3000 litres, muni d'un système d'évent (9) ;
- on place ledit conteneur (C), pendant tout le temps de son transport, pendant son stockage et pendant le soutirage de la crème de levure hors du conteneur (C) pour l'utilisation de celle-ci, dans une enceinte (16) dont on maintient la température à une température comprise entre -1°C et 10°C, de préférence entre 0 et 8°C, en particulier entre 0 et 5°C ;
- on prélève, par gravité et/ou par une pompe volumétrique (36), la crème de levure au voisinage du fond du conteneur (C) pour l'amener dans une ou des tuyauteries de distribution (29) de la crème de levure stabilisée pour son introduction dans un ou des pétrins (34) ; et
- on nettoie en place au moyen d'une solution de nettoyage au moins partiellement la ou les tuyauteries de distribution (29), après une période de temps déterminée.

2. Procédé suivant la revendication 1, **caractérisé par le fait que** le système d'évent (9) est muni d'un filtre empêchant l'entrée de contaminants.

3. Procédé suivant l'une quelconque des revendications 1 et 2, **caractérisé en ce que** le conteneur (C) est équipé :
- d'une tuyauterie de remplissage (42) amenant la crème de levure au voisinage du fond du conteneur (C),
- d'au moins une boule de nettoyage (23),
- d'une sonde de niveau (15),
- d'un système d'évent pour maintenir le conteneur (C) à pression atmosphérique (9),
- d'une vanne de vidange de niveau bas (C4).

4. Procédé suivant la revendication 3, **caractérisé par le fait que** la tuyauterie de remplissage (42) est sensiblement verticale, **par le fait qu'**elle est perforée (44) latéralement près du haut du conteneur (C) de manière à pouvoir servir d'évent, et **par le fait qu'**elle présente une ouverture (43) située à une distance de moins de 10 cm, de préférence de moins de 5 cm du fond du conteneur (C).

5. Procédé suivant la revendication 3 ou 4, **caractérisé par le fait que** le conteneur (C) est équipé d'au moins deux tuyauteries (42, 45, 46) perforées (44) latéralement près du haut du conteneur (C), de manière à pouvoir servir d'évent (9).

6. Procédé suivant la revendication 1, **caractérisé par le fait que** le conteneur (C) est une poche souple (5), de préférence jetable, autoportante ou de préférence sous forme d'une poche supportée par une armature recyclable.

7. Procédé suivant la revendication 6, **caractérisé par le fait que** le conteneur souple (5) est équipé d'un système (10) réutilisable formant évent (9) et sonde de niveau (15), et **par le fait qu'**il peut être vidangé complètement.

8. Procédé suivant l'une quelconque des revendications 3 à 5 et 7, **caractérisé par le fait que** la sonde de niveau (15) est munie d'une alarme de niveau bas.

9. Procédé suivant l'une quelconque des revendications 1 à 8, **caractérisée par le fait qu'**on utilise une pompe volumétrique (36) permettant une circulation lente de la crème de levure et une circulation rapide de la solution de nettoyage.

10. Procédé suivant la revendication 9, **caractérisé par le fait que** la pompe volumétrique (36) peut être réglée pour avoir une vitesse de circulation de la crème de levure comprise entre 0,5 et 2 m/s.

11. Procédé suivant la revendication 9 ou 10, **caractérisé par le fait que** la pompe volumétrique (36) peut être réglée pour avoir une vitesse de circulation de la solution de nettoyage supérieure ou égale à 2 m/s.

12. Procédé suivant l'une quelconque des revendications 1 à 11, **caractérisé par le fait que** la pompe volumétrique (36) est choisie dans le groupe suivant : pompes pneumatiques à membrane, pompes à lobes, pompes à vis excentrée ou queue de cochon.

13. Procédé suivant l'une quelconque des revendications précédentes, **caractérisé par le fait qu'**on détecte au moyen d'un témoin la détérioration de la qualité boulangère de la crème de levure stabilisée.

14. Procédé suivant l'une quelconque des revendications précédentes, **caractérisé par le fait qu'**on utilise la pompe volumétrique (36) pour le prélèvement de crème de levure hors du conteneur (C) et pour l'amener dans une boucle de circulation (B) de crème de levure comprenant ladite pompe volumétrique (36), **par le fait qu'**on prélève en un ou des points de prélèvement de la boucle (B) de la crème de levure pour alimenter un ou des pétrins (34), et qu'on retourne la quantité de crème stabilisée non prélevée au susdit ou aux susdits points de prélèvement vers le conteneur (C).

15. Procédé suivant la revendication 14, **caractérisé en ce que** le conteneur est équipé :
- d'une tuyauterie de remplissage (42),
- d'au moins une boule de nettoyage (23),
- d'une sonde de niveau (15),
- d'un système d'évent (9) pour maintenir le conteneur (C) à pression atmosphérique,
- d'une vanne de vidange de niveau bas (C4), ladite tuyauterie de remplissage (42) arrivant suffisamment à proximité du fond du conteneur (C) de manière à ce que quand la crème de levure est mise en circulation fermée, il n'y ait pas formation de mousse.

16. Procédé suivant l'une quelconque des revendications 14 et 15, **caractérisé par le fait qu'**on ramène la crème de levure dans le conteneur (C) par une tuyauterie (42) présentant une ouverture (43) située au-dessus du fond de celui-ci, à une distance de moins de 10 cm, de préférence de moins de 5 cm dudit fond.

17. Procédé suivant l'une quelconque des revendications 14 à 16, **caractérisé par le fait qu'**on refroidit la crème dé levure au moyen d'un échangeur de chaleur (27), avant de ramener la crème de levure dans le conteneur (C).

18. Procédé suivant l'une quelconque des revendications 14 à 17, **caractérisé par le fait que** chaque partie de la boucle (B) de crème de levure est auto-vidangeable, c'est-à-dire chaque partie de la boucle (B) a un point bas par lequel elle peut être vidée entièrement.

19. Procédé suivant l'une quelconque des revendications 1 à 18, **caractérisé par le fait que** les tuyauteries de distribution (29) de crème de levure peuvent être mises en circuit fermé sur un réservoir (25) contenant la solution de nettoyage, cette dernière circulant grâce à la pompe volumétrique (36) permettant de véhiculer ladite crème de levure.

20. Procédé suivant l'une quelconque des revendications 1 à 19, **caractérisé par le fait qu'**on utilise pour la stabilisation de la crème de levure un additif alimentaire sélectionné dans la famille des homopolysaccharides, éventuellement modifiés, de préférence dans le groupe des amidons et des dérivés de la cellulose.

21. Procédé suivant la revendication 20, **caractérisé par le fait qu'**on utilise comme additif alimentaire pour la stabilisation de la crème de levure une dispersion homogène d'un gel d'amidon modifié.

22. Procédé suivant l'une des revendications 20 et 21, **caractérisé par le fait qu'**on utilise comme additif alimentaire pour la stabilisation de la crème de levure 0,5 % à 2,0 % en poids de la crème de levure d'un amidon thermiquement et/ou chimiquement modifié, et de préférence entre 0,5 % et 1,5 % en poids de la crème de levure d'un amidon chimiquement modifié.

23. Procédé suivant l'une quelconque des revendications 20 à 22, **caractérisé par le fait qu'**on utilise comme additif alimentaire pour la stabilisation de la crème de levure de l'adipate de diamidon acétylé répondant à la définition de l'amidon modifié E 1422 dans la nomenclature européenne à raison de 0,5 % à 1,5 %, de préférence entre 0,8 % et 1,0 % en poids de la crème de levure.

24. Procédé suivant la revendication 20, **caractérisé par le fait qu'**on utilise comme additif alimentaire pour la stabilisation de la crème de levure une Dispersion d'un dérivé de la cellulose, de préférence de carboxyméthylcellulose.

25. Procédé suivant la revendication 20, **caractérisé par le fait qu'**on utilise comme additif alimentaire pour la stabilisation de la crème de levure une carboxyméthylcellulose haute viscosité à raison de 0,1 % à 0,3 % en poids de la crème de levure.

26. Conteneur (C) pour la mise en oeuvre du procédé suivant l'une quelconque des revendications 1 à 25, ledit conteneur présentant :
- un volume compris entre 100 et 3000 litres,
- un système d'évent (9), tel que le conteneur (C) reste toujours à la pression atmosphérique,
- une vanne (C4) située au voisinage du fond du conteneur (C) pour permettre le soutirage de la crème de levure,
- au moins une boule de nettoyage (23), et
- une tuyauterie de remplissage (42) du conteneur (C), ladite tuyauterie (42) amenant la crème de levure au voisinage du fond du conteneur (C),
et dans lequel la tuyauterie de remplissage (42) est sensiblement verticale, présente une ouverture (43) située au-dessus du fond du conteneur (C), à une distance de moins de 10 cm, de préférence de moins de 5 cm dudit fond, et comporte au moins une perforation (44) latérale près du haut du conteneur (C), de manière à pouvoir servir d'évent (9).

27. Conteneur (C) suivant la revendication 26, **caractérisé par le fait qu'**il est muni d'un détecteur de niveau minimal (15) de crème de levure dans le conteneur (C), ledit détecteur émettant un signal d'alarme lorsque le détecteur détecte ledit niveau minimal.

28. Conteneur (C) suivant l'une quelconque des revendications 26 à 27, **caractérisé par le fait qu'**il comporte au moins un témoin de la détérioration de la qualité boulangère de la crème de levure stabilisée présente dans le conteneur (C).

29. Conteneur (C) suivant l'une quelconque des revendications 26 à 28, **caractérisé par le fait qu'**il comporte au moins deux tuyauteries (42, 45, 46) sensiblement verticales et perforées (44) à proximité du haut du conteneur (C), de manière à ce qu'au moins une tuyauterie (42, 45, 46) soit libre pour être reliée à l'extérieur du conteneur (C) et forme un évent (9), l'évent appartenant de préférence à l'un des types suivants : tube en U, crépine, filtre, filtre bactériologique.

30. Installation pour le conditionnement et l'utilisation, sous forme d'une suspension stable, d'une crème de levure stabilisée par l'emploi d'un additif alimentaire de la famille des épaississants et stabilisants alimentaires après une conservation tranquille et prolongée, ladite installation comportant :
- un conteneur (C) propre et quasiment stérile d'un volume compris entre environ 100 litres et 3000 litres et muni d'un système d'évent (9),
- une enceinte (16) définissant une chambre contenant le conteneur (C), la température de ladite chambre étant maintenue à une température comprise entre-1°C et 10°C,
- une ou des tuyauteries de distribution (29), et
- un ou des pétrins (34),
l'installation permettant le prélèvement par gravité de la crème de levure stabilisée au voisinage du fond du conteneur (C) pour l'amener dans la ou les tuyauteries de distribution (29) pour son introduction dans le ou les pétrins (34),
ladite installation permettant le nettoyage en place au moyen d'une solution de nettoyage au moins partiellement de la ou des tuyauteries de distribution (29).

31. Installation pour le conditionnement et l'utilisation, sous forme d'une suspension stable, d'une crème de levure stabilisée par l'emploi d'un additif alimentaire de la famille des épaississants et stabilisants alimentaires après une conservation tranquille et prolongée, installation comportant :
- un conteneur (C) propre et quasiment stérile d'un volume compris entre environ 100 litres et 3000 litres muni d'un système d'évent (9),
- une enceinte (16) définissant une chambre contenant le conteneur (C), la température de ladite chambre étant maintenue à une température comprise entre -1°C et 10°C,
- une ou des tuyauteries de distribution (29),
- une pompe volumétrique (36), et
- un ou des pétrins (34),
ladite pompe volumétrique permettant le prélèvement de la crème de levure stabilisée au voisinage du fond du conteneur (C) pour l'amener dans la ou les tuyauteries de distribution (29) pour son introduction dans le ou les pétrins (34),
ladite installation permettant le nettoyage en place au moyen d'une solution de nettoyage au moins partiellement de la ou des tuyauteries de distribution (29).

32. Installation suivant la revendication 31, **caractérisée en ce que** la pompe volumétrique (36) est choisie dans le groupe suivant : pompes pneumatiques à membrane, pompes à lobes, pompes à vis excentrée ou queue de cochon.

33. Installation suivant l'une quelconque des revendications 30 à 32, **caractérisée en ce qu'**elle comporte un dispositif de nettoyage pour le nettoyage en place au moyen d'une solution de nettoyage d'au moins une partie de la ou des tuyauteries de distribution (29).

34. Installation suivant la revendication 33, **caractérisée en ce que** le dispositif de nettoyage comporte un réservoir (25) contenant la solution de nettoyage.

35. Installation suivant l'une quelconque des revendications 30 à 34, **caractérisée en ce que** le système d'évent (9) est muni d'un filtre empêchant l'entrée de contaminants.

36. Installation suivant l'une quelconque des revendications 30 à 35, **caractérisée en ce que** le conteneur (C) est équipé :
- d'une tuyauterie de remplissage (42) du conteneur (C), ladite tuyauterie (42) étant apte à amener la crème de levure au voisinage du fond du conteneur (C),
- d'au moins une boule de nettoyage (23),
- d'une sonde de niveau (15),
- d'un système d'évent apte à maintenir le conteneur (C) à pression atmosphérique (9),
- d'une vanne de vidange de niveau bas (C4).

37. Installation suivant la revendication 36, **caractérisée en ce que** la tuyauterie de remplissage (42) est sensiblement verticale, par le fait qu'elle est perforée (44) latéralement près du haut du conteneur de manière à pouvoir servir d'évent, et par le fait qu'elle présente une ouverture (43) située à une distance de moins de 10 cm, de préférence de moins de 5 cm du fond du conteneur (C).

38. Installation suivant l'une quelconque des revendications 36 et 37, **caractérisée en ce que** le conteneur (C) est équipé d'au moins deux tuyauteries (42, 45, 46) perforées (44) latéralement près du haut du conteneur (C), de manière à pouvoir servir d'évent (9).

39. Installation pour la mise en oeuvre du procédé suivant l'une quelconque des revendication 1 à 25, ladite installation comprenant :
- une enceinte (16) définissant une chambre adaptée pour contenir au moins un conteneur propre et quasiment stérile (C), d'un volume compris entre environ 100 litres et 3000 litres, de préférence entre 200 litres et 3000 litres, muni d'un système d'évent (9), ladite enceinte (16) étant munie d'un dispositif pour maintenir la température de la chambre entre -1°C et 10°C, de préférence entre 0 et 8°C, en particulier entre 0 et 5°C ;
- une boucle de circulation (B) pour prélever de la crème de levure au voisinage du fond du conteneur (C) et pour amener la crème de levure vers un ou des points de prélèvement de la boucle (B) où de la crème de levure peut être soutirée pour un pétrin (34), ladite boucle (B) se terminant par une canalisation (52) permettant de ramener la crème de levure non prélevée dans le conteneur (C) après son passage dans la boucle (B) ;
- une pompe volumétrique (36) pour prélever de la crème de levure du conteneur (C) et pour refouler la crème de levure dans la boucle de circulation (B), ladite pompe (36) étant adaptée, d'une part, pour assurer l'écoulement de la crème de levure dans la boucle (B) à une vitesse comprise entre 0,5 et 2 m/s de manière à éviter tout échauffement de la crème de levure, et d'autre part, pour assurer une circulation efficace d'une solution de nettoyage dans ladite boucle de circulation (B), et
- un réservoir (25) pouvant être relié à la boucle de circulation par un ensemble de raccords mobiles ou tout autre dispositif prévu à cet effet et permettant le nettoyage en place en circuit fermé de l'ensemble des tuyauteries servant à la circulation de la crème de levure.

40. Installation suivant la revendication 39, **caractérisée par le fait que** la boucle de circulation (B) de la crème de levure comprend un échangeur de chaleur (27) permettant le refroidissement de la crème de levure avant son retour dans le conteneur (C).

41. Installation suivant la revendication 39 ou 40, **caractérisée par le fait qu'**elle comprend un système d'arrêt automatique de la pompe (36) lorsqu'un niveau minimal de levure dans le conteneur (C) est détecté.

42. Installation suivant l'une quelconque des revendications 39 à 41, **caractérisée par le fait que** chaque portion de la boucle de circulation (B) de la crème de levure est auto-vidangeable, c'est-à-dire peut se vider complètement par gravité.

43. Installation suivant l'une quelconque des revendications 39 à 42, **caractérisée par le fait que** l'enceinte (16) est adaptée pour contenir deux conteneurs (C, C_{RES}) de crème de levure stabilisée, un premier (C) étant monté sur la boucle de circulation (B) pour le soutirage de la crème de levure, tandis que le deuxième (C_{RES}) est en réserve.

44. Installation suivant la revendication 43, **caractérisée par le fait qu'**elle comporte un premier conteneur (C) et un deuxième conteneur (C_{RES}), lesdits premier et deuxième conteneurs (C, C_{RES}) pouvant chacun être reliés par un système de raccords, de préférence souples, et de vannes (109, 110, 113, 114) ou de vannes 3 voies d'une part, à une canalisation (100) alimentant la pompe volumétrique (36), et, d'autre part, à une tuyauterie d'alimentation (103, 104, 111, 112) du conteneur (C, C_{RES}) recevant la quantité de crème non prélevée, et **par le fait qu'**elle comporte un système de commande (130) desdites vannes (109, 110, 113, 114), de manière à ce que, lorsqu'un niveau minimal de crème de levure dans le conteneur (C, C_{RES}) en soutirage est détecté, le dispositif de commande (130) manoeuvre lesdites vannes (109, 110, 113, 114) pour que l'autre conteneur (C_{RES}, C) soit mis en utilisation sur la boucle de circulation (B) de la crème de levure.

45. Installation suivant l'une quelconque des revendications 39 à 44, **caractérisée par le fait que** la boucle de circulation (B) est prévue de manière à être mise automatiquement en circulation soit sur le conteneur de crème de levure (C), soit sur le réservoir (25) permettant le nettoyage en place.

46. Installation suivant l'une quelconque des revendications 30 à 45, **caractérisée en ce qu'**elle comporte un dispositif de dosage (32).

47. Installation suivant la revendication 46, **caractérisée en ce que** le dispositif de dosage comporte une ou des vannes électromagnétiques de dosage (32, 54).

48. Installation suivant l'une quelconque des revendications 46 et 47, **caractérisée en ce que** le dispositif de dosage comporte un débitmètre (31).

## Claims

1. Process for packaging and using a yeast cream after tranquil and prolonged storage, in the form of a stable suspension,
in which:
- the yeast cream is stabilized using a food additive from the family of food thickeners and stabilizers;
- the stabilized yeast cream cooled to below 10°C is packaged in a clean and virtually sterile container (C) with a volume of between about 100 litres and 3000 litres and preferably between 200 litres and 3000 litres, fitted with a vent system (9);
- the said container (C) is placed, throughout the period of its transportation, during its storage and during the removal of the yeast cream from the container (C) in order to use the said cream, in an enclosure (16) which is maintained at a temperature of between -1°C and 10°C, preferably between 0 and 8°C and in particular between 0 and 5°C;
- the yeast cream is collected, by gravity and/or by means of a positive-displacement pump (36), in the region of the bottom of the container (C) and is conveyed in one or more pipes (29) for distributing the stabilized yeast cream in order to introduce it into one or more dough mixers (34); and
- the distribution pipe(s) (29) is (are) at least partially cleaned in place using a cleaning solution, after a given period of time.

2. Process according to Claim 1, **characterized in that** the vent system (9) is fitted with a filter for preventing the inlet of contaminants.

3. Process according to either of Claims 1 and 2, **characterized in that** the container (C) is equipped
- with a filling pipe (42) for conveying the yeast cream to the bottom of the container (C),
- with at least one cleaning ball (23),
- with a level sensor (15),
- with a vent system (9) for maintaining the container (C) at atmospheric pressure,
- with a low-level emptying valve (C4).

4. Process according to Claim 3, **characterized in that** the filling pipe (42) is substantially vertical, due to the fact that it is perforated (44) laterally close to the top of the container (C) so as to be able to serve as a vent, and due to the fact that it has an aperture (43) located at a distance of less than 10 cm and preferably of less than 5 cm from the bottom of the container (C).

5. Process according to Claim 3 or 4, **characterized in that** the container (C) is equipped with at least two pipes (42, 45, 46) perforated (44) laterally close to the top of the container (C) so as to be able to serve as a vent (9).

6. Process according to Claim 1, **characterized in that** the container (C) is a flexible bag (5), preferably a disposable bag, which is self supporting or, preferably, in the form of a bag supported by a recyclable reinforcement.

7. Process according to Claim 6, **characterized in that** the flexible container (5) is equipped with a reusable system (10) forming a vent (9) and a level sensor (15), and **in that** it may be emptied completely.

8. Process according to any one of Claims 3 to 5 and 7, 5 **characterized in that** the level sensor (15) is fitted with a low-level alarm.

9. Process according to any one of Claims 1 to 8, **characterized in that** a positive-displacement pump (36) allowing slow circulation of the yeast cream and fast circulation of the cleaning solution is used.

10. Process according to Claim 9, **characterized in that** the positive-displacement pump (36) may be adjusted so as to have a yeast cream circulation speed of between 0.5 and 2 m/s.

11. Process according to Claim 9 or 10, **characterized in that** the positive-displacement pump (36) may be adjusted so as to have a cleaning solution circulation speed of greater than or equal to 2 m/s.

12. Process according to any one of Claims 1 to 11, **characterized in that** the positive-displacement pump (36) is chosen from the following group: pneumatic diaphragm pumps, lobe pumps, processing cavity pumps and Archimedean screw pumps.

13. Process according to any one of the preceding claims, **characterized in that** the deterioration in the baking quality of the stabilized yeast cream is detected by means of a control.

14. Process according to any one of the preceding claims, **characterized in that** the positive-displacement pump (36) is used to take yeast cream from the container (C) and convey it to a yeast cream circulation loop (B) comprising the said positive-displacement pump (36), **in that** yeast cream is taken from one or more collection points of the loop (B) and fed into one or more dough mixers (34), and **in that** the amount of stabilized cream not taken from the said collection point(s) is returned to the container (C).

15. Process according to Claim 14, **characterized in that** the container (C) is equipped
- with a filling pipe (42),
- with at least one cleaning ball (23),
- with a level sensor (15),
- with a vent system (9) for maintaining the container (C) at atmospheric pressure,
- with a low-level emptying valve (C4),
the said filling pipe (42) arriving sufficiently close to the bottom of the container (C) so that when the yeast cream is placed in closed circulation, there is no formation of foam.

16. Process according to either of Claims 14 and 15, **characterized in that** the yeast cream is returned to the container (C) via a pipe (42) which has an aperture (43) located above the bottom of the said container, at a distance of less than 10 cm and preferably of less than 5 cm from the said bottom.

17. Process according to any one of Claims 14 to 16, **characterized in that** the yeast cream is cooled using a heat exchanger (27) before returning the yeast cream to the container (C).

18. Process according to any one of Claims 14 to 17, **characterized in that** each part of the yeast cream loop (B) can be self-emptied, that is to say that each part of the loop (B) has a bottom point via which it may be entirely emptied.

19. Process according to any one of Claims 1 to 18, **characterized in that** the yeast cream distribution pipes (29) may be placed in a closed circuit on a tank (25) containing the cleaning solution, this solution circulating by means of the positive-displacement pump (36) allowing the said yeast cream to be conveyed.

20. Process according to any one of Claims 1 to 19, **characterized in that** a food additive selected from the family of optionally modified homopolysaccharides and preferably from the group of starches and cellulose derivatives is used to stabilize the yeast cream.

21. Process according to Claim 20, **characterized in that** a homogeneous dispersion of a modified starch gel is used as food additive to stabilize the yeast cream.

22. Process according to either of Claims 20 and 21, **characterized in that** 0.5% to 2.0%, by weight of the yeast cream, of a thermally and/or chemically modified starch, and preferably between 0.5% and 1.5%, by weight of the yeast cream, of a chemically modified starch, is used as food additive to stabilize the yeast cream.

23. Process according to any one of Claims 20 to 22, **characterized in that** acetylated starch adipate corresponding to the definition of the modified starch E 1422 in the European nomenclature is used as food additive to stabilize the yeast cream, in a proportion of from 0.5% to 1.5% and preferably between 0.8% and 1.0% by weight of the yeast cream.

24. Process according to Claim 20, **characterized in that** a dispersion of a cellulose derivative, preferably carboxymethylcellulose, is used as food additive to stabilize the yeast cream.

25. Process according to Claim 20, **characterized in that** a high-viscosity carboxymethylcellulose is used as food additive to stabilize the yeast cream, in a proportion of from 0.1% to 0.3% by weight of the yeast cream.

26. Container (C) for carrying out the process according to any one of Claims 1 to 25, the said container having :
- a volume of between 100 and 3000 litres,
- a vent system (9), such that the container (C) always remains at atmospheric pressure,
- a valve (C4) located in the region of the bottom of the container (C) to allow the yeast cream to be removed,
- at least one cleaning ball (23), and
- a pipe (42) for filling the container (C), the said pipe (42) conveying the yeast cream to the bottom of the container (C),
and in which the filling pipe (42) is substantially vertical, has an aperture (43) located above the bottom of the container (C), at a distance of less than 10 cm and preferably of less than 5 cm from the said bottom, and comprises at least one lateral perforation (44) close to the top of the container (C) so as to be able to serve as a vent (9).

27. Container (C) according to Claim 26, **characterized in that** it is fitted with a detector (15) for detecting a minimum level of yeast cream in the container (C), the said detector emitting an alarm signal when the detector detects the said minimum level.

28. Container (C) according to any one of Claims 26 to 27, **characterized in that** it comprises at least one control of the deterioration in the baking quality of the stabilized yeast cream present in the container (C).

29. Container (C) according to any one of Claims 26 to 28, **characterized in that** it comprises at least two substantially vertical pipes (42, 45, 46) perforated (44) close to the top of the container (C), such that at least one pipe (42, 45, 46) is free to be connected to the outside of the container (C) and forms a vent (9), the vent preferably belonging to one of the following types: U-tube, strainer, filter, bacteriological filter.

30. Plant for packaging and using, in the form of a stable suspension, a yeast cream stabilized using a food additive from the family of food thickeners and stabilizers, after a tranquil and prolonged storage, the said plant comprising:
- a clean and virtually sterile container (C) with a volume of between about 100 litres and 3000 litres and fitted with a vent system (9),
- an enclosure (16) defining a chamber containing the container (C), the said chamber being maintained at a temperature of between -1°C and 10°C,
- one or more distribution pipes (29), and
- one or more dough mixers (34),
the said plant enabling collecting by gravity stabilized yeast cream in the region of the bottom of the container (C) to introduce it into the dough mixer(s) (34).
the said plant enabling the at least partial cleaning in place of the distribution pipe(s) (29) using a cleaning solution.

31. Plant for packaging and using, in the form of a stable suspension, a yeast cream stabilized using a food additive from the family of food thickeners and stabilizers, after a tranquil and prolonged storage, the said plant comprising:
- a clean and virtually sterile container (C) with a volume of between about 100 litres and 3000 litres and fitted with a vent system (9),
- an enclosure (16) defining a chamber containing the container (C), the said chamber being maintained at a temperature of between -1°C and 10°C,
- one or more distribution pipes (29),
- a positive-displacement pump (36), and
- one or more dough mixers (34),
the said positive-displacement pump being capable of collecting stabilized yeast cream in the region of the bottom of the container (C) in order to convey it to the distribution pipe(s) (29) to introduce it into the dough mixer (s) (34),
the said plant enabling the at least partial cleaning in place of the distribution pipe(s) (29) using a cleaning solution.

32. Plant according to Claim 31, **characterized in that** the positive-displacement pump (36) is chosen from the following group: pneumatic diaphragm pumps, lobe pumps, processing cavity pumps and Archimedean screw pumps.

33. Plant according to any one of Claims 30 to 32, **characterized in that** it comprises a cleaning device for the in-place cleaning of at least some of the distribution pipe(s) (29) using a cleaning solution.

34. Plant according to Claim 33, **characterized in that** the cleaning device comprises a tank (25) containing the cleaning solution.

35. Plant according to any one of Claims 30 to 34, **characterized in that** the vent system (9) is fitted with a filter for preventing the inlet of contaminants.

36. Plant according to any one of Claims 30 to 35, **characterized in that** the container (C) is equipped:
- with a pipe (42) for filling the container (C), the said pipe (42) being capable of conveying the yeast cream to the bottom of the container (C),
- with at least one cleaning ball (23),
- with a level sensor (15),
- with a vent system (9) capable of maintaining the container (C) at atmospheric pressure,
- with a low-level emptying valve (C4).

37. Plant according to Claim 36, **characterized in that** the filling pipe (42) is substantially vertical, due to the fact that it is perforated (44) laterally close to the top of the container so as to be able to serve as a vent, and due to the fact that it has an aperture (43) located at a distance of less than 10 cm and preferably of less than 5 cm from the bottom of the container (C).

38. Plant according to either of Claims 36 and 37, **characterized in that** the container (C) is equipped with at least two pipes (42, 45, 46) perforated (44) laterally close to the top of the container (C) so as to be able to serve as a vent (9).

39. Plant for carrying out the process according to any one of Claims 1 to 25, the said plant comprising:
- an enclosure (16) defining a chamber which is suitable for containing at least one clean and virtually sterile container (C), the said enclosure (16) being fitted with a device for maintaining the chamber at a temperature of between -1°C and 10°C, preferably between 0 and 8°C and in particular between 0 and 5°C;
- a circulation loop (B) for collecting cream yeast in the region of the bottom of the container (C) and for conveying the yeast cream to one or more collection points of the loop (B) at which yeast cream may be removed for a dough mixer (34), the said loop ending with a channel (52) for returning to the container (C) the yeast cream which is not taken, after it has passed through the loop (B) ;
- a positive-displacement pump (36) for taking yeast cream from the container (C) and for delivering the yeast cream back into the circulation loop (B), the said pump (36) being suitable, firstly, for ensuring that the yeast cream in the loop (B) flows at a speed of between 0.5 and 2 m/s so as to avoid any heating of the yeast cream, and secondly, for ensuring an efficient circulation of a cleaning solution in the said circulation loop (B), and
- a tank (25) which may be connected to the circulation loop via a set of movable connections or any other device designed for this purpose which allows the in-place cleaning in the closed circuit of all of the pipes used for circulating the yeast cream.

40. Plant according to Claim 39, **characterized in that** the yeast cream circulation loop (B) comprises a heat exchanger (27) for cooling the yeast cream before it is returned to the container (C).

41. Plant according to Claim 39 or 40, **characterized in that** it comprises a system for automatically stopping the pump (36) when a minimum level of yeast in the container (C) is detected.

42. Plant according to any one of Claims 39 to 41, **characterized in that** each portion of the yeast cream circulation loop (B) can be self-emptied, that is to say that it can be completely emptied by gravity.

43. Plant according to any one of Claims 39 to 42, **characterized in that** the enclosure (16) is suitable for containing two stabilized yeast cream containers (C, C_{RES}), a first (C) being mounted on the circulation loop (B) for removing yeast cream, while the second (C_{RES}) is in reserve.

44. Plant according to Claim 43, **characterized in that** it comprises a first container (C) and a second container (C_{RES}), the said first and second containers (C, C_{RES}) each possibly being connected, via a system of connections, which are preferably flexible, and of valves (109, 110, 113, 114) or of 3-way valves, on the one hand to a channel (100) feeding the positive-displacement pump (36), and on the other hand to a feed pipe (103, 104, 111, 112) of the container (C, C_{RES}) receiving the amount of cream not taken, and **in that** it comprises a system (130) for controlling the said valves (109, 110, 113, 114) such that, when a minimum level of yeast cream in the container (C, C_{RES}) is detected during collection, the control device (130) actuates the said valves (109, 110, 113, 114) so that the other container (C_{RES}, C) is placed in service on the yeast cream circulation loop (B).

45. Plant according to any one of Claims 39 to 44, **characterized in that** the circulation loop (B) is designed so as to be placed in circulation automatically either on the yeast cream container (C) or on the tank (25) for in-place cleaning.

46. Plant according to any one of Claims 30 to 45, **characterized in that** it comprises a metering device (32).

47. Plant according to Claim 46, **characterized in that** the metering device comprises one or more electromagnetic metering valves (32, 54).

48. Plant according to either of Claims 46 and 47, **characterized in that** the metering device comprises a flow meter (31).

## Patentansprüche

1. Verfahren zur Konditionierung und zur Verwendung einer Hefecreme nach einer ruhigen und langen Aufbewahrung in Form einer stabilen Suspension, wobei:
- die Hefecreme durch die Verwendung eines Lebensmitteladditivs, das zu der Gattung der Lebensmittel-Verdickungsmittel und -Stabilisatoren gehört; stabilisiert wird,
- die auf weniger als 10°Cgekühlte stabilisierte Hefecreme konditioniert wird, und in einem sauberen fast sterilen Gefäß (C), mit einem Fassungsvermögen zwischen etwa 100 und 3000 Liter, vorzugsweise zwischen 200 und 3000 Liter, versehen mit einem Lüftungssystem (9);
- das Gefäß (C), während des ganzen Transports, während seiner Lagerung und während die Hefecreme aus dem Gefäß (C) zur Verwendung entnommen wird, in einem Fach (16) untergebracht wird, dessen Temperatur zwischen -1°C und 10°C, vorzugsweise zwischen 0 und 8°C, insbesondere zwischen 0 und 5°C gehalten wird;
- die Hefecreme in der Nähe des Gefäßbodens (C) durch Schwerkraft und/oder durch eine Zumeßpumpe (36) entnommen wird, damit sie in ein Rohr oder in Röhren (29) zur Verteilung der stabilisierten Hefecreme eintritt, und in ein Knetwerk oder in Knetwerke (34) eingeführt wird; und
- nach einer vorgegebenen Zeitspanne das Verteilerrohr oder die Verteilerröhren (29) mittels einer Reinigungslösung mindestens teilweise an Ort und Stelle gereinigt wird oder werden.

2. Verfahren gemäß Anspruch 1, **dadurch gekennzeichnet, dass** das Lüftungssystem (9) mit einem Filter versehen ist, das den Eintritt von Kontaminierungsmitteln verhindert.

3. Verfahren gemäß einem der Ansprüche 1 und 2, **dadurch gekennzeichnet, dass** das Gefäß (C) mit
- einer Entleerungsrohrleitung (42), welche die Hefecreme in die Nähe des Bodens des Gefäßes (C) bringt,
- mindestens einer Reinigungskugel (23),
- einer Niveausonde (15)
- einem Lüftungssystem (9) zur Beibehaltung des atmosphärischen Druckes im Gefäß (C),
- einem niedrig angeordneten Entleerungsventil (C4)
ausgestattet ist.

4. Verfahren gemäß Anspruch 3, **dadurch gekennzeichnet, dass** die Entleerungsrohrleitung (42) im wesentlichen vertikal ist, dass sie seitlich in der Nähe des Oberteils des Gefäßes (C) perforiert ist (44), damit sie als Lüftungssystem dient, und dass sie eine Öffnung (43) aufweist, die weniger als 10 cm, vorzugsweise weniger als 5 cm vom Boden des Gefäßes (C) entfernt ist.

5. Verfahren gemäß Anspruch 3 oder 4, **dadurch gekennzeichnet, dass** das Gefäß (C) mit mindestens zwei Rohrleitungen (42, 45, 46) ausgestattet ist, welche in der Nähe des Oberteils des Gefäßes (C) seitlich perforiert sind (44), um als Luftungssystem (9) zu dienen.

6. Verfahren gemäß Anspruch 1, **dadurch gekennzeichnet, dass** das Gefäß (C) eine verformbare Tasche (5) ist, vorzugsweise eine Wegwerftasche, selbsttragend oder vorzugsweise in Form einer durch eine wieder verwendbare Vorrichtung aufgenommen Tasche.

7. Verfahren gemäß Anspruch 6, **dadurch gekennzeichnet, dass** das verformbare Gefäß (5) mit einem wieder verwendbaren System (10) in Form eines Lüftungssystems (9) und einer Niveausonde (15) ausgestattet ist, und dass es vollständig entleert werden kann.

8. Verfahren gemäß einem der Ansprüche 3 bis 5 und 7, **dadurch gekennzeichnet dass**, die Niveausonde (15) auf Niedrigniveau ansprechenden Alarmvorrichtung versehen ist.

9. Verfahren gemäß einem der Ansprüche 1 bis 8, **dadurch gekennzeichnet, dass** eine Zumeßpumpe (36) eingesetzt wird, um eine langsame Zirkulation der Hefecreme und eine schnelle Zirkulation der Reinigungslösung zu erlauben.

10. Verfahren gemäß Anspruch 9, **dadurch gekennzeichnet, dass** die Zumeßpumpe (36) derart geregelt werden kann, dass eine Zirkulationsgeschwindigkeit der Hefecreme zwischen 0,5 und 2 m/s erreicht wind.

11. Verfahren gemäß Anspruch 9 oder 10, **dadurch gekennzeichnet, dass** die Zumeßpumpe (36) derart geregelt werden kann, dass eine Zirkulationsgeschwindigkeit der Reinigungslösung größer oder gleich 2 m/s erreicht wird.

12. Verfahren gemäß einem der Ansprüche 1 bis 11, **dadurch gekennzeichnet, dass** die Zumeßpumpe (36) aus einer der folgenden Gruppen ausgewählt wird: pneumatische Membranpumpen, Pumpen mit Flügeln, Exzenter-Schneckenpumpen oder mit Gewindedom.

13. Verfahren gemäß einem der vorigen Ansprüche, **dadurch gekennzeichnet, dass** die Verschlechterung der Backqualität der stabilisierten Hefecreme mittels einer Anzeige festgestellt wird.

14. Verfahren gemäß einem der vorigen Ansprüche, **dadurch gekennzeichnet, dass** die Zumeßpumpe (36) zur Entnahme der Hefecreme aus dem Gefäß (C) und zu ihrer Einführung in eine Zirkulationsschleife (B) der Hefecreme, welche die Zumeßpumpe (36) beinhaltet, eingesetzt wird, dass Hefecreme an einer Stelle oder mehreren Stellen der Schleife (B) entnommen wird, um ein Knetwerk oder Knetwerke (34) zu speisen, und dass die Quantität der eingesetzten stabilisierten Creme, die an der genannten Stelle oder den genannten Stellen nicht entnommen wurde, zu dem Gefäß (C) zurückgefördert wird.

15. Verfahren gemäß Anspruch 14, **dadurch gekennzeichnet, dass** das Gefäß (C) mit
- einer Entleerungsrohrleitung (42),
- mindestens einer Reinigungskugel (23),
- einer Niveausonde (15),
- einem Lüftungssystem (9) zur Beibehaltung des atmosphärische Druckes im Gefäß (C),
- einem niedrig angeordneten Entleerungsventil (C4)
ausgestattet ist, wobei die genannte Entleerungsrohrleitung (42) genügend nah an dem Boden des Gefäßes (C) liegt, damit keine Schaumbildung entsteht, wenn die Hefecreme im geschlossenen Zirkulationskreis in Bewegung gesetzt wird.

16. Verfahren gemäß einem der Ansprüche 14 und 15, **dadurch gekennzeichnet, dass** die Hefecreme in das Gefäß (C) mittels einer Rohrleitung (42) zurückgefördert wird, die eine Öffnung (43) über dessen Boden aufweist, die vom Boden weniger als 10 cm, vorzugsweise weniger als 5 cm entfernt ist.

17. Verfahren gemäß einem der Ansprüche 14 bis 16, **dadurch gekennzeichnet, dass** die Hefecreme mittels eines Wärmetauschers (27) gekühlt wird, bevor die Hefecreme in das Gefäß (C) zurückgefördert wird.

18. Verfahren gemäß einem der Ansprüche 14 bis 17, **dadurch gekennzeichnet, dass** jedes Teil der Schleife (B) der Hefecreme selbstentleerbar ist, d.h. dass jedes Teil der Schleife (B) eine niedrige Stelle aufweist, an welcher sie vollständig entleerbar ist.

19. Verfahren gemäß einem der Ansprüche 1 bis 18, **dadurch gekennzeichnet, dass** die Verteilerröhren (29) der Hefecreme auf einem eine Reinigungslösung enthaltenden Behälter (25) auf geschlossenen Zirkulationskreis geschaltet werden können, wobei der Transport der Hefecreme über die Zumeßpumpe (36) mittels der Reinigungslösung erfolgt.

20. Verfahren gemäß einem der Ansprüche 1 bis 19, **dadurch gekennzeichnet, dass** für die Stabilisierung der Hefecreme ein aus der Gattung der Homopolysacchariden, eventuell modifiziertes, vorzugsweise aus der Gruppe der Stärken und der Cellulosenderivaten ausgewähltes Lebensmitteladditiv verwendet wird.

21. Verfahren gemäß Anspruch 20, **dadurch gekennzeichnet, dass** eine homogene Dispersion eines modifizierten Stärkegels als Lebensmitteladditiv zur Stabilisierung der Hefecreme verwendet wird.

22. Verfahren gemäß einem der Ansprüche 20 und 21, **dadurch gekennzeichnet, dass** als Lebensmitteladditiv für die Stabilisierung der Hefecreme 0,5 Gew.% bis 2,0 Gew.% der Hefecreme einer thermisch und/oder chemisch modifizierten Stärke, und vorzugsweise zwischen 0,5 Gew.% und 1,5 Gew.% der Hefecreme einer chemisch modifizierten Stärke verwendet wird.

23. Verfahren gemäß einem der Ansprüche 20 bis 22, **dadurch gekennzeichnet, dass** als Lebensmitteladditiv für die Stabilisierung der Hefecreme acetyliertes Adipinat eines Diamids entsprechend der Definition der modifizierten Stärke E 1422 gemäß der europäischen Nomenklatur bei 0,5 Gew.% bis 1,5 Gew.%, vorzugsweise zwischen 0,8 Gew.% und 1,0 Gew.% der Hefecreme verwendet wird.

24. Verfahren gemäß Anspruch 20, **dadurch gekennzeichnet, dass** als Lebensmitteladditiv zur Stabilisierung der Hefecreme eine Dispersion eines Cellulosenderivates, vorzugsweise von Carboxymethylcellulose verwendet wird.

25. Verfahren gemäß Anspruch 20, **dadurch gekennzeichnet, dass** als Lebensmitteladditiv zur Stabilisierung der Hefecreme eine Carboxymethylcellulose mit hoher Viskosität bei 0,1 Gew.% bis 0,3 Gew.% der Hefecreme verwendet wird.

26. Gefäß (C) zur Anwendung des Verfahrens gemäß einem der Ansprüche 1 bis 25, wobei das Gefäß (C):
- ein Fassungsvermögen zwischen 100 und 3000 Liter,
- ein Lüftungssystem (9), zur Beibehaltung des atmosphärischen Druckes,
- ein in der Nähe des Gefäßbodens (C) angebrachtes Ventil (C4), um die Entnahme der Hefecreme zu ermöglichen,
- mindestens eine Reinigungskugel (23), und
- eine Entleerungsrohrleitung (42) des Gefäßes (C), welche Entleerungsrohrleitung (42) die Hefecreme in die Nähe des Gefäßbodens (C) bringt,
aufweist,
wobei die Entleerungsrohrleitung (42) im wesentlichen vertikal verläuft, eine über dem Gefäßboden angebrachte Öffnung (43) aufweist, die weniger als 10 cm, vorzugsweise weniger als 5 cm vom Boden entfernt ist, und mindestens eine seitliche Perforierung (44) nahe dem Oberteil des Gefäßes (C) aufweist, welche als Lüftungssystem (9) dient.

27. Gefäß (C) gemäß Anspruch 26, **dadurch gekennzeichnet, dass** es mit einem Detektor (15) des im Gefäß (C) erreichten Mindestniveaus der Hefecreme ausgestattet ist, wobei der Detektor beim Feststellen des Mindestniveaus ein Alarmsignal sendet.

28. Gefäß (C) gemäß einem der Ansprüche 26 bis 27, **dadurch gekennzeichnet, dass** es mindestens eine Anzeige der Verschlechterung der Backqualität der im Gefäß (C) befindlichen stabilisierten Hefecreme umfaßt.

29. Gefäß (C) gemäß einem der Ansprüche 26 bis 28, **dadurch gekennzeichnet, dass** es mindestens zwei Rohrleitungen (42, 45, 46) beinhaltet, welche im wesentlichen vertikal und nahe dem Oberteil des Gefäßes (C) perforiert (44) sind, so dass mindestens eine Rohrleitung (42, 45, 46) frei ist, um außerhalb des Gefäßes (C) verbunden zu werden und ein Lüftungssystem (9) bildet, welches vorzugsweise zu einem der folgenden Typen gehört: Uförmiges Rohr, Schutzfilter, Filter, bakteriologischer Filter.

30. Anlage zur Konditionierung und Verwendung, in Form einer stabilen Suspension, einer Hefecreme, die durch die Verwendung eines Lebensmitteladditivs der Gattung der Verdickungsmittel und der Lebensmittelstabilisatoren nach ruhiger und langer Aufbewahrung stabilisiert wurde, wobei die Anlage
- ein sauberes und fast steriles Gefäß (C) mit einem Fassungsvermögen zwischen etwa 100 und 3000 Liter, ausgestattet mit einem Lüftungssystem (9),
- ein Fach (16) entsprechend einer das Gefäß (C) enthaltenden Kammer, wobei die Temperatur der Kammer zwischen -1°C und 10°C gehalten wird,
- ein Verteilerrohr oder Verteilerröhre (29) und
- ein Knetwerk oder Knetwerke (34),
aufweist, wobei die Anlage eine Entnahme der stabilisierten Hefecreme durch Schwerkraft nahe dem Gefäßboden (C) erlaubt, damit sie in ein Verteilerrohr oder in Verteilerröhre (29) eintritt, um einem Knetwerk oder Knetwerken (34) zugeführt zu werden,
wobei die Anlage die mindestens teilweise Reinigung des Verteilerrohrs oder der Verteilerröhren (29) an Ort und Stelle erlaubt.

31. Anlage zur Konditionierung und Verwendung, in Form einer stabilen Suspension, einer Hefecreme, die durch die Verwendung eines Lebensmitteladditivs der Gattung der Verdickungsmittel und der Lebensmittelstabilisatoren nach einer ruhigen und langen Aufbewahrung stabilisiert wurde, wobei die Anlage
- ein sauberes und fast steriles Gefäß (C) mit einem Fassungsvermögen zwischen etwa 100 und 3000 Liter, ausgestattet mit einem Lüftungssystem (9),
- ein Fach (16) entsprechend einer das Gefäß (C) enthaltenden Kammer, wobei die Temperatur der Kammer zwischen -1°C und 10°C gehalten wird,
- ein Verteilerrohr oder Verteilerröhre (29),
- eine Zumeßpumpe (36) und
- ein Knetwerk oder Knetwerke (34),
aufweist,
wobei die Zumeßpumpe eine Entnahme der stabilisierten Hefecreme nahe dem Gefäßboden (C) erlaubt, damit sie in ein Verteilerrohr oder in Verteilerröhre (29) eintritt, um einem Knetwerk oder Knetwerken (34) zugeführt zu werden,
wobei die Anlage die mindestens teilweise Reinigung des Verteilerrohrs oder der Verteilerröhren (29) an Ort und Stelle erlaubt.

32. Anlage gemäß Anspruch 31, **dadurch gekennzeichnet, dass** die Zumeßpumpe (36) aus der folgenden Gruppe ausgewählt wird: pneumatische Membranpumpen, Pumpen mit Flügeln, Exzenter-Schneckenpumpen oder mit Gewindedom.

33. Anlage gemäß einem der Ansprüche 30 bis 32, **dadurch gekennzeichnet, dass** sie eine Reinigungsvorrichtung zur Reinigung an Ort und Stelle mittels einer Reinigungslösung für mindestens ein Teil des Verteilungsrohrs oder der Verteilungsröhre (29) aufweist.

34. Anlage gemäß Anspruch 33, **dadurch gekennzeichnet, dass** die Reinigungsvorrichtung einen Behälter (25) aufweist, der die Reinigungslösung enthält.

35. Anlage gemäß einem der Ansprüche 30 bis 34, **dadurch gekennzeichnet, dass** das Lüftungssystem (9) mit einem Filter ausgestattet ist, das den Eintritt von Kontaminierungsmitteln verhindert.

36. Anlage gemäß einem der Ansprüche 30 bis 35, **dadurch gekennzeichnet, dass** das Gefäß (C) mit
- einer Entleerungsrohrleitung (42) zum Entleeren des Gefäßes (C), wobei die Rohrleitung die Hefecreme in die Nähe des Gefäßbodens (C) bringt,
- mindestens einer Reinigungskugel (23),
- einer Niveausonde (15),
- einem Lüftungssystem (9), zur Beibehaltung des atmosphärischen Druckes,
- inem niedrig angeordneten Entleerungsventil (C4)
ausgestattet ist.

37. Anlage gemäß Anspruch 36, **dadurch gekennzeichnet, dass** die Entleerungsrohrleitung (42) im wesentlichen vertikal ist, dass sie nahe des Oberteils des Gefäßes derart seitlich perforiert ist (44), dass sie als Lüftungssystem dient, und dass sie eine Öffnung (43) aufweist, die weniger als 10 cm, vorzugsweise weniger als 5 cm vom Boden des Gefäßes (C) entfernt ist.

38. Anlage gemäß einem der Ansprüche 36 und 37, **dadurch gekennzeichnet, dass** das Gefäß (C) mit mindestens zwei Rohrleitungen (42, 45, 46) ausgestattet ist, die in der Nähe des Oberteils des Gefäßes (C) seitlich perforiert (44) sind, um als Lüftungssystem (9) zu dienen.

39. Anlage zur Anwendung des Verfahrens gemäß einem der Ansprüche 1 bis 25, wobei die Anlage:
- ein Fach (16) entsprechend einer Kammer, die derart ausgestaltet ist, dass sie mindestens ein sauberes und fast steriles Gefäß (C) mit einem Fassungsvermögen zwischen etwa 100 und 3000 Liter, vorzugsweise zwischen 200 und 3000 Liter enthält, das ein Lüftungssystem (9) aufweist, wobei das Fach (16) mit einer Vorrichtung zur Beibehaltung der Temperatur der Kammer zwischen -1 °C und 10°C, vorzugsweise zwischen 0 und 8°C insbesondere zwischen 0 und 5°C versehen ist;
- eine Zirkulationsschleife (B) zur Entnahme der Hefecreme in der Nähe des Bodens des Gefäßes (C) und zur Führung der Hefecreme zu einer oder mehreren Entnahmestellen der Schleife (B), an welchen Hefecreme für ein Knetwerk (34) entnommen werden kann, welche Schleife (B) mit einer Rohrleitung (52) endet, die es ermöglicht, die im Gefäß nicht entnommene Hefecreme nach ihrem Durchgang durch die Schleife (B) in das Gefäß (C) zurückzufördern;
- eine Zumeßpumpe (36) zur Entnahme der Hefecreme aus dem Gefäß (C) und zum Zurückdrängen der Hefecreme in die Zirkulationsschleife (B), welche Pumpe (36) derart ausgestaltet ist, dass sie einerseits die Zirkulation der Hefecreme durch die Schleife (B) mit einer Geschwindigkeit zwischen 0,5 und 2 m/s sichert, um jede Erwärmung der Hefecreme zu vermeiden, und andererseits eine wirksame Zirkulation einer Reinigungslösung in die Zirkulationsschleife (B) sichert;
- einen Behälter (25), welcher mit der Zirkulationsschleife durch eine Anzahl von beweglichen Anschlüssen oder durch jede andere diesbezüglich vorgesehene Vorrichtung verbunden werden kann, wobei an Ort und Stelle im geschlossenen Zirkulationskreis eine Reinigung der gesamten Rohrleitungen ermöglicht wind, die zur Zirkulation der Hefecreme dienen,
aufweist.

40. Anlage gemäß Anspruch 39, **dadurch gekennzeichnet, dass** die Zirkulationsschleife (B) der Hefecreme einen Wärmetauscher (27) aufweist, der es ermöglicht die Hefecreme abzukühlen bevor sie in das Gefäß (C) zurückgefördert wird.

41. Anlage gemäß Anspruch 39 oder 40, **dadurch gekennzeichnet, dass** sie ein automatisches Sperrsystem aufweist, das die Pumpe (36) anhält, wenn ein Mindestniveau der Hefe im Gefäß (C) festgestellt wird.

42. Anlage gemäß einem der Ansprüche 39 bis 41, **dadurch gekennzeichnet, dass** jeder Abschnitt der Durchflußschleife (B) der Hefecreme selbstentleerbar ist, d.h. durch Schwerkraft vollständig entleerbar ist.

43. Anlage gemäß einem der Ansprüche 39 bis 42, **dadurch gekennzeichnet, dass** das Fach (16) derart ausgestaltet ist, das es zwei Gefässe (C, C_{RES}) mit stabilisierter Hefecreme beinhalten kann, wobei das erste Gefäß (C) auf der Zirkulationsschleife (B) zur Entnahme der Hefecreme angebracht ist, während das zweite (C_{RES}) in Reserve bleibt.

44. Anlage gemäß Anspruch 43, **dadurch gekennzeichnet, dass** sie ein erstes Gefäß (C) und ein zweites Gefäß (C_{RES}) beinhaltet, wobei beide genannte Gefässe (C, C_{RES}) jeweils das erste und das zweite durch ein System von vorzugsweise verformbaren Anschlüssen und von Ventilen (109, 110, 113, 114) oder Dreiwegventilen einerseits, mit einer die Zumeßpumpe (36) speisende Rohrleitung (100) verbunden werden können, und andererseits mit einer Zufuhrrohrleitung (103, 104, 111, 112) für das Gefäß (C, C_{RES}), welche die nicht entnommene Crememenge erhält, und dadurch, dass sie ein System (130) zur Steuerung der Ventile (109, 110, 113, 114) enthält, damit bei Feststellung eines Mindestniveaus der Hefecreme in dem Gefäß (C, C_{RES}) während der Entnahme, das Steuerungsystem (130) die Ventile (109, 110, 113, 114) derart steuert, dass das andere Gefäß (C_{RES}, C) auf der Zirkulationsschleife (B) der Hefecreme zum Einsatz gebracht wird.

45. Anlage gemäß einem der Ansprüche 39 bis 44, **dadurch gekennzeichnet, dass** die Zirkulationsschleife (B) vorgesehen ist, damit sie automatisch entweder auf das Gefäß mit der Hefecreme (C), oder auf den Behälter (25) zur Reinigung an Ort und Stelle aufgeschaltet wird.

46. Anlage gemäß einem der Ansprüche 30 bis 45, **dadurch gekennzeichnet, dass** sie eine Dosiervorrichtung (32) enthält.

47. Anlage gemäß Anspruch 46, **dadurch gekennzeichnet, dass** die Dosiervorrichtung ein elektromagnetisches Dosierventil oder elektromagnetische Dosierventile (32, 54) aufweist.

48. Anlage gemäß einem der Ansprüche 46 und 47, **dadurch gekennzeichnet, dass** die Dosiervorrichtung ein Durchflußmesser (31) aufweist.
